(19) Europäisches Patentamt
European Patent Office
Office européen des brevets

(11) **EP 4 224 486 B1**

(12) **EUROPEAN PATENT SPECIFICATION**

(45) Date of publication and mention
of the grant of the patent:
**05.08.2026 Bulletin 2026/32**

(21) Application number: **22382095.2**

(22) Date of filing: **04.02.2022**

(51) International Patent Classification (IPC):
**G16H 20/60** (2018.01)

(52) Cooperative Patent Classification (CPC):
**G16H 20/60;** Y02A 90/10

(54) **COMPUTER IMPLEMENTED METHOD FOR DESIGNING A DIET**

COMPUTERIMPLEMENTIERTES VERFAHREN ZUM ENTWURF EINER DIÄT

PROCÉDÉ MIS EN  UVRE PAR ORDINATEUR POUR CONCEVOIR UN RÉGIME ALIMENTAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**09.08.2023 Bulletin 2023/32**

(73) Proprietors:
• **Universidad del País Vasco/Euskal Herriko
Unibertsitatea
48940 LEIOA-VIZCAYA (ES)**
• **Universidad de Cantabria
39005 Santander (ES)**

(72) Inventors:
• **ODRIOZOLA MARTÍNEZ, Adrian
E-48940 LEIOA - VIZCAYA (ES)**
• **ÁLVAREZ-HERMS, Jesús
E-48940 LEIOA - VIZCAYA (ES)**
• **MERINO VALDEOLMILLOS, Ricardo
E-48940 LEIOA - VIZCAYA (ES)**
• **TIRNAUCA, Cristina
E-39005 Santander (ES)**
• **GONZÁLEZ BENITO, Adriana
E-48940 LEIOA - VIZCAYA (ES)**

(74) Representative: **ABG Intellectual Property Law,
S.L.
Avenida de Burgos, 16D
Edificio Euromor
28036 Madrid (ES)**

(56) References cited:
**JP-A- 2020 177 518    US-A1- 2020 381 089**

**Description**

**FIELD OF THE INVENTION**

The present invention relates to the field of methods for designing a personalized diet for a user. More particularly, the diet pursues to improve the user's gut microbiome composition.

**BACKGROUND OF THE INVENTION**

The gut microbiome is the diverse community of microorganisms living in our gut, with enormous impact in health, due to its fundamental role in regulating inflammation and metabolism, intestinal permeability, immune system, B and K group vitamins and neurotransmitters.

There are many elements that directly affect and are affected by the gut microbiome composition such as nutrition, physical activity, use of antibiotics, presence of pathogens and physical and mental stress level.

Therefore, disruptive factors such as a sedentary lifestyle and/or an unhealthy nutrition can alter the quantitative and qualitative composition of microbiome, shattering its healthy balance and fostering its transition into a state known as microbiome dysbiosis. Long-term microbiome dysbiosis is being associated with a degenerative cyclic process characterized by an increase in intestinal and systemic chronic inflammation, an increase in intestinal permeability, a reduction of immune and inflammatory response capacity, and thus, with a progressive reduction in the allostatic capacity of the individual to maintain the internal milieu within positive health limits against future disruptive factors.

Fortunately, due to recent advances in human fecal genetic analysis, determining the composition of gut microbiome can be done following a three-steps process: 1) a faeces DNA purification, which eliminates the rest of undigested carbohydrate, fiber, protein fat and inorganic components from the diet; 2) a DNA amplification by PCR of 16S rRNA taxonomic marker; and finally, 3) a bioinformatics DNA sequence analysis, which determines the composition and quantitative abundance of microorganisms. Thanks to these technological advances, a raising number of specific microbiome dysbiosis are increasingly associated with a variety of conditions such as obesity, diabetes, autism, psoriasis, kidney stones or even to more severe symptomatology in COVID-19 infection.

Promisingly, there is a booming growth in scientific evidence (in terms of associations and practical interventions) that show how to interrupt the negative cycle of microbiome dysbiosis associated with those different diseases.

In particular, studies that identify those disruptive factors and cause-effect relationships exhibit, in the same time, the consequences of decreasing the exposure to them to the minimum (recovery of microbiome balance and restoration of intestinal impermeability). Moreover, fecal microbiome transplants are being successfully used in the treatment of recurrent *Clostridioides* difficile infection, inflammatory bowel disease, especially in ulcerative colitis, and recently in systemic sclerosis.

In this context, reversion of specific microbiome dysbiosis by adhering to precision health principles represents a promising research area for new health-promoting applications regarding physical activity, regulation of circadian rhythms, stress management and precision nutrition

From a global perspective, precision nutrition seeks to integrate previous knowledge of individual microbiome, genome, physiology and habits. Its main aim is to determine individual nutritional requirements of prebiotics (bacterial food), probiotics (beneficial bacteria) and postbiotics (metabolites produced by bacteria) such as to supplement accordingly (preferentially by choosing those components that are naturally present in food) in order to optimize the allostatic capacity to tolerate and survive against specific disruptive factors with the minimum wear and tear of the organism.

The practical challenge of applying precision nutrition involves dealing with the following main aspects: 1) each complex trait or phenotype is associated with a specific microbiome composition, 2) certain bacteria can be diminished in some phenotypes and increased in other, 3) each bacterium can grow with specific combinations of prebiotics or food components, containing variable amounts of each prebiotic, and 4) specific bacteria can be naturally present in some food components.

JP2020177518 is a document close to the invention that describes a device and a program for selecting food to bacterium of intestinal bacterial flora. US 2020/381089 A1 describes a system and a method of data interpretation to provide recommendations to the user on the basis of his genetic data and data on the composition of gut microbiota.

Therefore, there is a need in the art of new methods that allow professionals to offer individualized support in choosing those combinations of nutriments that supply our organisms the highest benefits in terms of microbiome.

**SUMMARY OF THE INVENTION**

The present invention provides a solution for the aforementioned problems by a computer implemented-method for designing a diet for a user according to claim 1, a data processing system according to claim 14, a computer program according to claim 15, and a computer-readable medium according to claim 16. In dependent claims, preferred embodiments of the invention are defined.

In a first inventive aspect, the invention provides a computer implemented method for designing a diet for a user, the method comprising the following steps:

a) accessing a database, the database comprising

- a list of foods, each food comprising at least one food prebiotic, FP;
- a first list of bacterial species commonly found in the gut microbiome of subjects from the species to which the user belongs; and
- a list of at least one bacterium prebiotic, BP, consumed by each bacterium of the first list of bacterial species;

b) providing a second list of bacteria comprising the bacteria lacking in the user's gut microbiome and/or the bacteria present in a low quantity in the user's gut microbiome;

c) selecting a food, F; and for each bacterium, $B_i$, of the second list of bacteria; wherein i is an index from 1 to the number of bacteria of said second list:

I. obtaining, from the database, the list of at least one bacterium prebiotic BP consumed by the bacterium $B_i$; and
II. comparing the at least one food prebiotic FP of the food F with the list of at least one bacterium prebiotic BP, such that:

i. if the at least one food prebiotic FP totally matches with the at least one bacterium prebiotic BP of the list, assigning the food F with a maximum value predefined; or
ii. if none of the at least one food prebiotic FP matches with the at least one bacterium prebiotic BP of the list, assigning the food F with a minimum value predefined; or
iii. if a subset of the at least one food prebiotic FP matches with the at least one bacterium prebiotic BP of the list, assigning the food F with a value given by a partial sum of a harmonic series; wherein the number of elements of the harmonic series is equal to the number of food prebiotics of the subset;

d) generating a vector of growth promoting for the food F, wherein each element of the vector is the value assigned to the food F for each bacterium $B_i$ of the second list of bacteria;
e) repeating the previous steps for a plurality of foods;
f) calculating, for each food, the sum of the elements of its vector of growth promoting;
g) generating a ranking of foods from the food with the highest sum of the elements of its vector of growth promoting to the food with the lowest sum of the elements of its vector of growth promoting;
h) designing the diet so that it comprises the food or foods of the top position or positions of the ranking, being the total number of foods of the diet predefined.

The method of the first inventive aspect pursues designing a personalized diet for a user; in particular, a diet that will lead to an improvement in the user's gut microbiome composition.

Throughout all this document, "*diet*" will be understood as the sum of food consumed by a user or subject. "User or subject" as used herein, relates to any animal classified as mammal and includes but is not limited to domestic and farm animals, primates and humans, for example human beings, non-human primates, cows, horses, pigs, sheep, goats, dogs, cats or rodents. Preferably, the subject is a female or male human being of any race or age. In the context of the present invention, the subject is a subject who potentially suffers from gut dysbiosis. In another preferred embodiment, the user suffers obesity, diabetes, autism, psoriasis, kidney stones or COVID-19 infection.

"Gut microbiome" as used herein relates to a complex ecosystem that lives symbiotically with a human and has an essential role in digest function. It includes the microorganisms including bacteria, archaea and fungi that live in the digestive tracts of humans.

As mentioned before, an unhealthy nutrition can alter the quantitative and qualitative composition of the gut microbiome, having an enormous impact on the user's health due to its role in regulating inflammation, metabolism, intestinal permeability, and immune system, among others.

The personalized diet achieved by the present method is based on the relation between food components and the qualitative and quantitative composition of a user's gut microbiome.

The method requires continued access to a database with, at least, the following information:

- a list of foods, each food comprising at least one food prebiotic, FP;
- a first list of bacterial species commonly found in the gut microbiome of subjects from the species to which the user belongs; and

- a list of at least one bacterium prebiotic consumed by each bacterium of said first list.

"Food prebiotic or prebiotic" as used herein means non-digestible food substances that promote the growth of health beneficial micro-organisms and/or probiotics in the intestines. They are not broken down in the stomach and/or upper intestine or absorbed in the GI tract of the subject ingesting them, but they are fermented by the gastrointestinal microbiome and/or by probiotics. Prebiotics are typically thought of as carbohydrates of relatively short chain length. Prebiotics are like other carbohydrates that reach the cecum, such as non-starch polysaccharides, sugar alcohols, and resistant starch, in being substrates for fermentation. They are, however, distinctive in their selective effect on the microflora. To be effective, prebiotics must reach the cecum. Preferably, they may be selected from the group consisting of oligosaccharides, optionally containing fructose, galactose, mannose; dietary fibers, in particular soluble fibers, soy fibers; inulin; or mixtures thereof. Preferred prebiotics are fructo-oligosaccharides, galacto-oligosaccharides, isomalto-oligosaccharides, xylo-oligosaccharides, arabino-xylo oligosaccharides, mannan-oligosaccharides, oligosaccharides of soy, glycosylsucrose, lactosucrose, lactulose, palatinose-oligosaccharides, malto-oligosaccharides, gums and/or hydrolysates thereof, pectins and/or hydrolysates thereof, polyphenols, resveratrol, quercetin, raffinose, arabinoxylan arabinoxylanoligosaccharides, arabinogalactans, beta-glucans, tryptophan, resistant starch, fructooligosaccharides, inulin, pectin, galacto-oligosaccharides (GOS), beta-glucans, lignans, ellagitannins, curcumin, anthocyanins, and isoflavones.

Throughout all this document, *"the first list of bacterial species commonly found in the gut microbiome of subjects from the species to which the user belongs"* will be understood as a set of bacteria that can be present in the gut microbiome of any subject, whichever its species, that are beneficial for said subject's health.

Apart from said first lists of food and bacteria, as mentioned before, the method requires to count on information about the prebiotics that said bacteria consume. Therefore, *"bacterium prebiotic",* as used herein relates to the prebiotic that is consumed by a bacterium.

All this information comprised in the database is based on scientific evidence.

Note that this step of accessing a database is concurrent with the rest of the steps of the method; that is, the database can be accessed at each moment during the execution of the rest of the steps of the whole method.

Next, the method provides a second list of bacteria comprising the bacteria lacking in the user's gut microbiome and/or the bacteria present in a low quantity in the user's gut microbiome. Note that the bacteria of this second list are a subset of the bacteria of the first list of the database so it is possible to obtain the lists of bacterium prebiotic consumed by all the bacteria of the second list by accessing the database.

The terms *"low quantity"* in this context must be understood in the light of the scientific evidence, that is, a low quantity of bacteria in the user's gut microbiome is an insufficient number of bacteria for considering the gut microbiome as healthy. The quantification of bacteria can be performed using any method known in the art, such as CFU count, absorbance, microscopy, flow cytometry, Methylene Blue dye Reduction Test (MBRT) or Start Growth Method (SGT). Alternatively the Gut Microbiome Health Index (GMHI), a mathematical formula that determines the degree to which a gut microbiome profile reflects good or adverse health or the Microbiome Modulation Index (MMI) can be used in the present invention to determine the quantity of bacteria that perform beneficial functions for the human organism.

In a preferred manner, the second list of bacteria is created according to a fecal genetic analysis.

Next, the method continues with the selection of a food F and with an iterative process in which, for each of the i-th bacterium of the second list of the database, the following steps are carried out. Note that i is an index from 1 to the number of bacteria of the second list, without prejudice of being possible to use another different index notation.

Firstly, the method requires obtaining, from the database, the list of at least one bacterium prebiotic BP consumed by the bacterium $B_i$ and comparing said bacterium prebiotic or prebiotics, BP, with the at least one food prebiotic FP of the food F.

At this point, three options are available:

- The food prebiotic or prebiotics FP totally matches with the bacterium prebiotic or prebiotics BP. In this case, the food F is assigned with a maximum value predefined; for example, a value of one.
- None of the food prebiotic or prebiotics FP matches with the bacterium prebiotic or prebiotics BP. In this case, the food F is assigned with a minimum value predefined; for example, a value of zero.
- Part of the food prebiotics FP matches with the bacterium prebiotics BP. In this case, the value assigned to the food F will depend on the number of matching prebiotics according to a partial sum of a harmonic series. In particular, the number of elements of the harmonic series will be equal to the number of food prebiotics that matches with the bacterium prebiotics.
  In other words, if S is the intersection between the food prebiotics for the given food F and the list of bacterium prebiotics for the given bacterium $B_i$, then the number of elements of the harmonic series is equal to the cardinality of the set S, being the partial sums of the harmonic series preferably as follows:

$$1 + \frac{1}{2} + \frac{1}{3} + \cdots$$

The rationale behind using this logarithmic growth in the previous formula is based on the assumption that a particular bacterium can grow in the presence of several prebiotics, but the relative importance of including a new one diminishes progressively: without nutrients, these microorganisms cannot grow; but, they can survive with different combinations of prebiotics. Therefore, the inclusion of additional prebiotics improves growth but it is not critical.

The iterative process finishes once the food F has been assigned with a certain value for each of the bacterium of the second list. At this point, for the food F, a vector of growth promoting is generated so that each element of the vector corresponds with the value assigned to the food F for each bacterium $B_i$.

Next, the previous steps c) and d), concurrently with step a), are repeated for different kind of foods, resulting in a set of different vectors of growth promoting.

A ranking of foods is then performed according to the sum of elements of the vectors of growth promoting. The ranking begins with the foods with the vector of growth promoting with the highest sum of elements to the food with the vector of growth promoting with the lowest sum of elements.

In this way, once it has been quantified the extent to which each food contributes to improving a certain bacterium that the user microbiome lacks (or only has in a small quantity), the method calculates the total contribution of each food to the user's microbiome by simply adding together all these amounts. The food ranking will be determined by this total value: the higher the value, the better the selected food in the sense that it covers a wider range of lacking and/or low-quantity bacteria. Thus, in an advantageous manner, the method allows to visualize both the individual effect and the cumulative total effect of the top foods of the ranking that are good candidates to be part of the personalized diet.

Finally, the method ends designing the diet so that it comprises the food of the best position in the ranking. The diet can be designed with more than one food, all of them being placed in the top positions of the ranking.

It must be noted that the scientific evidence regarding associations between microbiome and nutrition is growing exponentially in the last years, continuously discovering new connections between specific bacteria and prebiotic components. Thus, aiming to minimize the error of not yet discovered interactions, the method of the invention targets all possible existing prebiotics and not only those that are currently associated with the bacteria. Besides, it must be noted that the values assigned to a food with the method of the invention are comparable across the whole spectrum of bacteria, i.e., a food that shares the same number of prebiotics with two different bacteria will have the same value.

Therefore, the first inventive aspect proposes an innovative precision nutrition bioinformatic tool: a food recommender method for promoting gut microbiome balance based on the individual microbiome. It allows to determine individual nutritional requirements of prebiotics by the ingestion of components naturally presented in food. Optionally, the diet could be designed to further comprise nutrition supplements.

Advantageously, the method of the invention boosts individual well-being by carefully selecting the sort of daily food that is most suitable for the user, avoiding a diet founded on the consumption of great quantities of prebiotic supplements. Also note that ingesting a high quantity of different type of nutriments would successfully nourish the user's body, but this scenario is neither healthy nor realistic. Conversely, the method designs a diet that can be followed by the users in real conditions without exerting a high effort.

Another advantage of the method is that it is adapted just for a user's target bacteria (lacking bacteria or low-quantity bacteria). This allows to specialize the design of the diet using just the foods that have the impact being pursuit; e.g. weight control, emotional balance, intestinal health, increased metabolism rate, energy intake, etc.

In a particular embodiment, the value assigned to each food is normalized between zero and one.

In a particular embodiment, the database further comprises a list of at least one food consumed by at least one of the bacterium of the first list of bacterial species; and wherein the method further comprises the following sub-steps, being said sub-steps carried out before step c.I)

c1) obtaining, from the database, the list of at least one food consumed by the bacterium $B_i$, if any;
c2) if the food F is comprised in the list of at least one food consumed by the bacterium $B_i$, assigning the food F with the maximum value predefined; or
c3) if the food F is not comprised in the list of at least one food consumed by the bacterium $B_i$ or if there is not a list of at least one food consumed by the bacterium $B_i$ in the database, going to step c.I) of the method.

In this embodiment, before analyzing which prebiotics of the food F are consumed by a specific bacterium $B_i$, the method firstly checks if there is an association between said bacterium $B_i$ and the food F that is endorsed directly by the scientific community. In such cases, the method grants the maximum value predefined to said food F for this bacterium $B_i$.

To achieve this, this embodiment requires that the database further comprises a list with at least one food consumed by at least one of the bacterium of the first list of bacterial species.

In this way, the method requires a step of obtaining, from the database, the list of at least one food that the bacterium consumes, if any, and checking if the food F is comprised in this list of foods. If so, the food F is given the maximum value; for example, a normalized value of 1. After this, the method continues in step c1) if there are further bacteria of the second list to be analyzed or in step d) if all the bacteria have been analyzed for the given food F.

Otherwise, i.e. when direct evidence is lacking, the intensity of the association between a bacterium $B_i$ and the food F is calculated through the prebiotics that F contains and $B_i$ consumes (again, according to scientific evidence). This means that, if the food F is not comprised in the list of foods consumed by the bacterium $B_i$, or if there is not a list with the foods consumed by the bacterium $B_i$ in the database, further checks are carried out by the method in order to assign a value to the food F; that is, the method continues with the step c.I).

In a particular embodiment, the database further comprises a variable that defines, for each bacterium prebiotic BP, its capacity for promoting growth in the gut of each bacterium of the first list; and wherein the value assigned to the food F in step cII.iii) is further based on said variable.

According to the current scientific evidence, the effect in the gut bacterial growth of each bacterium prebiotic can be different.

This embodiment pursues to adjust the value assigned to a food F, for the bacterium $B_i$, depending on the capacity that its food prebiotic or prebiotics have to promote the growth of said bacterium $B_i$ in the user's gut microbiome. Note that this capacity is equal to the capacity of its corresponding bacterium prebiotic BP, which is accessible in the database. Advantageously, the method takes into account the real interactions of the food prebiotics and the bacteria in the gut microbiome of the user.

In an example, if a food comprises a subset of food prebiotics FP that partially matches with the list of bacterium prebiotics BP consumed by the bacterium $B_i$, the value assigned to the food F takes into account a weighting factor for each food prebiotic of the subset, depending on its capacity to promote the growth of the bacterium $B_i$ (which is the same as the capacity of its matching bacterium prebiotic BP detailed in the database).

In a more particular embodiment, said variable is a binary variable which can have a first value which indicates that the bacterium prebiotic BP is a high growth promoter for the bacterium or a second value which indicates that the bacterium prebiotic BP is a low growth promoter for the bacterium.

In this embodiment, in the database, there is a classification (for each bacterium of the first list) of the bacterium prebiotics according to the variable that defines their capacity for promoting the growth of the bacterium in the gut microbiome of a subject. Said classification is binary, defining two different groups: high growth promoter prebiotics or low growth promoter prebiotics. Said classification is performed according to the scientific evidence.

In an example, if a food comprises a subset of food prebiotics FP that partially matches with the list of bacterium prebiotics BP consumed by the bacterium $B_i$, the value assigned to the food F takes into account a weighting factor applied to each food prebiotic of the subset, depending on if the corresponding bacterium prebiotic is high or low growth promoter. Note that this weighting factor (for this example of the particular embodiment) only can take one of two different values.

In a particular embodiment, the database further comprises the quantity in which each food prebiotic, FP, is present in each food; and wherein the value assigned to the food F in step cII.iii) is further based on said quantities in which the at least one food prebiotic FP is present in said food F.

According to the current scientific evidence regarding the quantity of food prebiotics in a food, the effect of each food prebiotic in the gut bacterial growth can be different.

This embodiment pursues to adjust the value assigned to a food F, for the bacterium $B_i$, depending on the quantities in which the at least one food prebiotic FP is present in said food F. Advantageously, the method takes into account the real interactions of the food prebiotics and the bacteria in the gut microbiome of the user.

In an example, if a food comprises a subset of food prebiotics that partially matches with the list of bacterium prebiotics consumed by the bacterium Bi, the value assigned to the food F takes into account a weighting factor applied to each food prebiotic of the subset, depending on the quantity in which it is present in the food F.

In a particular embodiment, the value assigned to the food F in step c2.iii) is performed according to the following equation:

$$Value\_food(n) = \begin{cases} 0, & if\ n = 0 \\ \frac{1}{2} * max.\ value\ predefined, & if\ n = \frac{1}{2} \\ \left(\frac{1}{2} + w \sum_{i=2}^{2n} \frac{1}{i}\right) * max.\ value\ predefined, & otherwise \end{cases},$$

wherein w is a coefficient calculated as follows:

$$w = \frac{1}{2} / \left(\sum_{i=2}^{2N} \frac{1}{i}\right),$$

being $N$ the total number of bacterium prebiotics contained in the database and $n = \frac{1}{2} * n_l + 1 * n_h$ , being $n_l$ the number of low growth promoter prebiotics and $n_h$ is the number of high growth promoter prebiotics, wherein

a low growth promoter food prebiotic is a food prebiotic that is present in the food F in a quantity below a first predefined threshold and/or a food prebiotic for which the variable that defines its capacity for promoting growth of the bacterium $B_i$ in the gut microbiome is below a second predefined threshold; and
a high growth promoter food prebiotic is a food prebiotic that is present in the food F in a quantity above or equal the first predefined threshold and/or a food prebiotic for which the variable that defines its capacity for promoting growth of the bacterium $B_i$ in the gut microbiome is above or equal to the second predefined threshold.

According to the current scientific evidence regarding the quantity of prebiotics in each food component and/or the effect in the gut bacterial growth, the prebiotics can be classified in each food as either *"high growth promoters"* or *"low growth promoters"* to adjust the values assigned to the foods to the real interactions in gut microbiome.

The distinction between high growth promoters and low growth promoters is performed in case the quantity of prebiotic is above (or equal) or below a predefined first threshold, being said first threshold established according to the scientific evidence. This information is comprised in the database.

Additionally or alternatively, the distinction between high growth promoters and low growth promoters is based on the variable that defines its capacity for promoting growth of the bacterium under study in the gut microbiome of the subject. In particular, if the variable is below a second predefined threshold, the food prebiotic is classified as low growth promoter and if the variable is above or equal to the second predefined threshold, the food prebiotic is classified as high growth promoter. This second predefined threshold is stablished according to the scientific evidence. Note that this capacity is the same as the capacity that its corresponding bacterium prebiotic has to promote the growth of a bacterium, being said information comprised in the database.

It must be noted that the previous formula describes a modified partial sum of a harmonic series. The modification is performed to take into account if the food prebiotics are low or high growth promoters which, in turn, depends on the capacity of each food prebiotic to promote the growth of a bacterium in the gut microbiome of a user and/or on the quantity in which said food prebiotic is present in the food.

In this way, taking into account if the food prebiotics are high or low growth promoters, the value assigned to the food F is given by said previous formula. Advantageously, the method is more adapted to the reality and thus the diet designed will be more efficient for the improvement of the user's gut microbiome.

In a particular embodiment, the method further comprises the following sub-steps:

h1) calculating the Euclidean distance between the vector of growth promoting of the food or foods of the diet and each of the vectors of growth promoting of the rest of the foods, if any;
h2) designing the diet so that it further comprises the foods for which the distances estimated are the biggest ones, being the total number of foods of the diet predetermined.

In this embodiment, the diet is designed not only with the best food or foods (top foods) of the ranking but also with the foods that better fit with said top foods. In this way, this embodiment pursues to find the combination of foods that feed as many target bacteria as possible. This advantageously allows to design a diverse diet for the user's daily food intake without compromising too much on the effects it has on the user's microbiome.

To achieve this best combinations of foods, the method further comprises the steps of calculating the Euclidean distance between the vector of growth promoting of the foods on the top of the ranking and the rest of foods evaluated, if any. The diet is then completed with the foods for which the distances estimated are the biggest ones. The total number of foods of the diet is previously predetermined.

In a particular embodiment, the calculation of the Euclidean distances of sub-step h1) is performed using an agglomerative hierarchical clustering method. In a more particular embodiment, the agglomerative hierarchical clustering method is an Unweighted Pair Group Method with Arithmetic mean (UPGMA).

It must be also noted that once a list containing target bacteria that is either missing or insufficient in a user's gut microbiome and the score that quantifies the influence of different foods on each of these microorganisms is determined, obtaining the food or foods that maximizes the benefits (from a microbiomial point of view) using a fixed yet small number of foods is a highly complex combinatorial problem: there are more than 160000 possibilities to form groups of three foods from a set of 100, whereas exploring all combinations of four foods is slightly under 4000000 (the complexity also grows with the size of the set of foods).

To mitigate this problem, the method of the invention makes use of data mining tools. The unsupervised learning techniques used advantageously attacks said problem in a very transparent fashion: the models built are understandable, interpretable, reliable, and efficient.

The method uses an agglomerative (bottom-up) hierarchical clustering method; in particular, a method called UPGMA (unweighted pair group method with arithmetic mean), having as inputs the vectors of growth promoting of each food for the user.

This algorithm, used in bioinformatics for the creation of phenograms, produces rooted dendrograms and requires a constant-rate assumption (that is, it assumes an ultrametric tree in which the distances from the root to every branch tip are equal). Alternative linkage schemes generate different dendrograms because of the formula used to compute the inter-cluster distance. In the case of the UPGMA, the distance between two clusters Ci and Cj involves considering the size of their subclusters:

$$D\left(C_i, C_j\right) = \frac{n_{i1}\, d\left(C_{i1}, C_j\right) + \; n_{i2}\, d\left(C_{i2}, C_j\right)}{n_{i1} + n_{i2}}$$

where $C_{i1}$ and $C_{i2}$ are the two clusters that join to form $C_i$ and $n_{i1}$ and $n_{i2}$ are their respective cardinalities.

An agglomerative clustering method starts by assigning each point to an individual cluster (so the number of clusters is initially equal to the number of points), and then proceeds by grouping together those clusters that are at the smallest intercluster distance. The algorithm finishes when all the points are grouped in only one big cluster. The final result can be easily interpreted by reading its corresponding dendrogram.

Foods that share the same vector representation (similar foods) are the first to be grouped together, and those that belong to groups that are farther apart in this high-dimensional space (foods that complement each other) will be among the last to be joined in a cluster. The experimental results demonstrate that picking up ingredients from distinct groups almost always leads to more complete diets.

In a particular embodiment, the foods are selected from a group consisting of Açaí, Brown algae, Corn Hazelnuts, Onion, Sarda, Agave, Brussels sprouts, Corn bran, Honey, Passion fruit, Sea buckthorn, Almonds, Buckwheat, Cucumber, Hypericum perforatum, Peanuts, Sesame, Amaranth, Capers, Curcuma longa, Integral rice, Pear Sorghum, Apples, Carrots, Dark chocolate, Jerusalem artichoke, Peppers, Sorghum bran, Apricot, Cashew nuts, Date, Kiwi, Pineapple, Soy, Artichoke, Cassava, Echinacea purpurea, Kuding tea, Pistachio, Spelt, Asparagus, Cauliflower, Egg, Larch Plantains, Spinach, Avocado, Celery, Eggplant, Leek, Plums, Spirulina alga, Bamboo shoots, Cheese, Endives, Lentils, Pomegranate, Strawberries, Bananas, Cherries, Feijoa, Longan, Potato, Sugar cane, Barley, Chestnuts, Fermented soy milk, Lotus seeds, Pumpkin, Sunflower, Barley bran, Chia seeds, Figs, Mango, Quince, Beer, Chicken, Garlic, Milk, Quinoa, Sweet potato, Beer yeast, Chickpeas, Ginkgo biloba, Millet, Radishes, Tomato, Beetroot, Chicory, Goji, Berries, Millet bran, Raspberries, Tuna, Black tea, Citrus, Gooseberries, Mushrooms-fungi, Red wine, Turkey, Blackberries, Cocoa powder, Grapes, Oat bran, Rice bran, Walnuts, Blueberries, Coconut, Green Tea, Oats, Rye, Watercress, Broad beans, Codonopsis pilosula, Green beans, Olive oil, Rye bran, Wheat, Broccoli, Coffee, Green peas, Olives, Salmon, Wheat bran, White lupin, Yacon, Yam and any combination thereof.

In a particular embodiment, the food prebiotics and the bacterium prebiotics are selected from a group consisting of xylooligosaccharides (XOS), resistant starch, fructooligosaccharides (FOS), inulin, pectin, polyphenols, resveratrol, quercetin, raffinose, arabinoxylan (AX), arabinoxylan oligosaccharides (AXOS), arabinogalactans, galacto-oligosaccharides (GOS), beta-glucans, tryptophan, mannan-oligosaccharides (MOS), lignans, ellagitannins, curcumin, anthocyanins, isoflavones, and any combination thereof.

In a particular embodiment, the bacteria of the database are selected from a group consisting of *Bacillus, Eubacterium rectale, Barnesiella, Faecalibacterium prausnitzii, Bifidobacterium, Lactobacillus acidophilus, Butyrivibrio, Lactobacillus brevis, Lachnospira, Lactobacillus delbrueckii, Leuconostoc, Lactobacillus casei, Oscillospira, Lactobacillus fermentum, Weisella, Lactobacillus paracasei, Bacillus subtilis, Lactobacillus plantarum, Bifidobacterium adolescentis, Lactobacillus reuteri, Bifidobacterium animalis, Lactobacillus rhamnosus, Bifidobacterium bifidum, Lactobacillus salivarius, Bifidobacterium longum, Leuconostoc mesenteroides, Collinsella aerofaciens, Roseburia hominis, Coprococcus catus, Roseburia inulinivorans, Enterococcus faecium, Selenomonas ruminantium, Eubacterium hallii, Streptococcus thermophiles, Anaerostipes, Anaerotruncus, Bacteroides, Butyricimonas, Gemmiger, Haemophilus, Lactococcus, Oscillibacter, Parabacteroides, Phascolarctobacterium, Subdoligranulum, Akkermansia muciniphila, Anaerostipes hadrus, Lactobacillus, Roseburia, Anaerotruncus colihominis, Bacteroides uniformis, Bacteroides vulgatus, Christensenella minuta, Clostridium butyricum, Clostridium leptum, Coprococcus, Coprococcus eutactus, Lactococcus lactis, Oxolobacter formigenes, Roseburia faecis, Roseburia intestinalis, Roseburia inulinivorans,* and any combination thereof.

In these previous embodiments, a list of foods, prebiotics and bacteria are presented without prejudice of being possible to count on further foods, prebiotics and bacteria that are suitable in the context of the invention.

In a second inventive aspect, the invention provides a data processing system comprising means for carrying out the steps of the method of the first inventive aspect.

In a third inventive aspect, the invention provides a computer program comprising instructions that, when the program is executed by a computer, cause the computer to carry out the steps of the method of the first inventive aspect.

In a fourth inventive aspect, the invention provides a computer-readable medium comprising instructions that, when executed by a computer, cause the computer to carry out the steps of the method of the first inventive aspect.

It is also described a method for increasing the richness of the gut microbiome in a user comprising

(i) selecting a diet for the user by the method according to the first inventive aspect, and
(ii) administering the diet selected in step (i) to the user for a sufficient amount of time to effect a change in the gut microbiome.

It is also described a method for increasing the content of a probiotic within the gut microbiome in a user comprising

(i) selecting a diet for the user by the method according to the fist inventive aspect, wherein the second list of bacteria provided in step b) consist of a single bacterium that coincides with probiotic whose contents are to be increased and
(ii) administering the diet selected in step (i) to the user for a sufficient amount of time to effect a change in the gut microbiome.

## BRIEF DESCRIPTION OF THE DRAWINGS

Figures 1a-1c. Flowcharts according to two different embodiments of the invention.
Figure 2. The graph of function F that estimates to what extent prebiotics contribute to the growth of bacteria.
Figure 3. Top 20 food ingredients.
Figure 4. Hierarchical clustering of the top 60 food ingredients

## DETAILED DESCRIPTION OF THE INVENTION

For the set of figures, it will be understood the following definitions for the terms mentioned below:
"Diet" as used herein to the sum of food consumed by a subject. In a preferred embodiment the foods of the diet are selected from the group consisting of Açaí, Brown algae, Corn Hazelnuts, Onion, Sarda, Agave, Brussels sprouts, Corn bran, Honey, Passion fruit, Sea buckthorn, Almonds, Buckwheat, Cucumber, Hypericum perforatum, Peanuts, Sesame, Amaranth, Capers, Curcuma longa, Integral rice, Pear Sorghum, Apples, Carrots, Dark chocolate, Jerusalem artichoke, Peppers, Sorghum bran, Apricot, Cashew nuts, Date, Kiwi, Pineapple, Soy, Artichoke, Cassava, Echinacea purpurea, Kuding tea, Pistachio, Spelt, Asparagus, Cauliflower, Egg, Larch Plantains, Spinach, Avocado, Celery, Eggplant, Leek, Plums, Spirulina alga, Bamboo shoots, Cheese, Endives, Lentils, Pomegranate, Strawberries, Bananas, Cherries, Feijoa, Longan, Potato, Sugar cane, Barley, Chestnuts, Fermented soy milk, Lotus seeds, Pumpkin, Sunflower, Barley bran, Chia seeds, Figs, Mango, Quince, Beer, Chicken, Garlic, Milk, Quinoa, Sweet potato, Beer yeast, Chickpeas, Ginkgo biloba, Millet, Radishes, Tomato, Beetroot, Chicory, Goji, Berries, Millet bran, Raspberries, Tuna, Black tea, Citrus, Gooseberries, Mushrooms-fungi, Red wine, Turkey, Blackberries, Cocoa powder, Grapes, Oat bran, Rice bran, Walnuts, Blueberries, Coconut, Green Tea, Oats, Rye, Watercress, Broad beans, Codonopsis pilosula, Green beans, Olive oil, Rye bran, Wheat, Broccoli, Coffee, Green peas, Olives, Salmon, Wheat bran, White lupin, Yacon, Yam and any combination thereof

"User or subject" as used herein, relates to any animal classified as mammal and includes but is not limited to domestic and farm animals, primates and humans, for example human beings, non-human primates, cows, horses, pigs, sheep, goats, dogs, cats or rodents. Preferably, the subject is a female or male human being of any race or age. In the context of the present invention, the subject is a subject who potentially suffers from gut dysbiosis. In another preferred embodiment, the user suffers obesity, diabetes, autism, psoriasis, kidney stones or COVID-19 infection.

"Gut dysbiosis", as used herein relates to a disruption to the microbiome homeostasis caused by an imbalance in the microflora of the gut, changes in their functional composition and metabolic activities, or a shift in their local distribution.

"Gut microbiome" as used herein relates to a complex ecosystem that lives symbiotically with a human and has an essential role in digest function. It includes the microorganisms including bacteria, archaea and fungi that live in the digestive tracts of humans

"Food prebiotic or prebiotic" as used herein means non-digestible food substances that promote the growth of health beneficial micro-organisms and/or probiotics in the intestines. They are not broken down in the stomach and/or upper intestine or absorbed in the GI tract of the person ingesting them, but they are fermented by the gastrointestinal microbiome and/or by probiotics. Prebiotics are typically thought of as carbohydrates of relatively short chain length. Prebiotics are like other carbohydrates that reach the cecum, such as non-starch polysaccharides, sugar alcohols, and resistant starch, in being substrates for fermentation. They are, however, distinctive in their selective effect on the microflora. To be effective, prebiotics must reach the cecum. Preferably, they may be selected from the group consisting of oligosaccharides, optionally containing fructose, galactose, mannose; dietary fibers, in particular soluble fibers, soy fibers; inulin; or mixtures thereof. Preferred prebiotics are fructo-oligosaccharides, galacto-oligosaccharides, isomalto-oligosaccharides, xylo-oligosaccharides, arabino-xylo oligosaccharides, mannan-oligosaccharides, oligosaccharides of soy, glycosylsucrose, lactosucrose, lactulose, palatinose-oligosaccharides, malto-oligosaccharides, gums and/or hydrolysates thereof, pectins and/or hydrolysates thereof, polyphenols, resveratrol, quercetin, raffinose, arabinoxylan arabinoxylanoligosaccharides, arabinogalactans, beta-glucans, tryptophan, resistant starch, fructooligosaccharides, inulin, pectin, galacto-oligosaccharides (GOS), beta-glucans, lignans, ellagitannins, curcumin, anthocyanins, and isoflavones.

"Bacterium prebiotic", as used herein relates to the prebiotic which is consumed by a bacterium.

"Probiotic", as used herein relates to bacteria that perform beneficial functions for the human organism. Among the probiotic bacteria, Bifidobacteria species are the most prominent. Bifidobacteria species, when in live and viable form, stimulate the immune system and exert a competitive exclusion of pathogenic and putrefactive bacteria, reduce the amounts of ammonia and cholesterol in the blood, and promote absorption of minerals. In addition, Bifidobacteria have been suggested to exert a preventive action against colon cancer, by reducing the activity of some enzymes that convert procarcinogen substances into carcinogen substances. It is believed that the probiotic bacteria exert their effects in a synergistic manner to curtail and retard the growth of pathogenic/detrimental bacteria of the gut. Illustrative non-limitative examples of probiotics are *Bacillus, Eubacterium rectale, Barnesiella, Faecalibacterium prausnitzii, Bifidobacterium, Lactobacillus acidophilus, Butyrivibrio, Lactobacillus brevis, Lachnospira, Lactobacillus delbrueckii, Leuconostoc, Lactobacillus casei, Oscillospira, Lactobacillus fermentum, Weisella, Lactobacillus paracasei, Bacillus subtilis, Lactobacillus plantarum, Bifidobacterium adolescentis, Lactobacillus reuteri, Bifidobacterium animalis, Lactobacillus rhamnosus, Bifidobacterium bifidum, Lactobacillus salivarius, Bifidobacterium longum, Leuconostoc mesenteroides, Collinsella aerofaciens, Roseburia hominis, Coprococcus catus, Roseburia inulinivorans, Enterococcus faecium, Selenomonas ruminantium, Eubacterium hallii, Streptococcus thermophiles, Anaerostipes, Anaerotruncus, Bacteroides, Butyricimonas, Gemmiger, Haemophilus, Lactococcus, Oscillibacter, Parabacteroides, Phascolarctobacterium, Subdoligranulum, Akkermansia muciniphila, Anaerostipes hadrus, Lactobacillus, Roseburia, Anaerotruncus colihominis, Bacteroides uniformis, Bacteroides vulgatus, Christensenella minuta, Clostridium butyricum, Clostridium leptum, Coprococcus, Coprococcus eutactus, Lactococcus lactis, Oxolobacter formigenes, Roseburia faecis, Roseburia intestinalis, Roseburia inulinivorans.* In a preferred embodiment the prebiotic is *Akkermansia muciniphila, Eubacterium hallii, Lactococcus* genera or *Lactobacillus* genera.

Computer implemented method of the invention

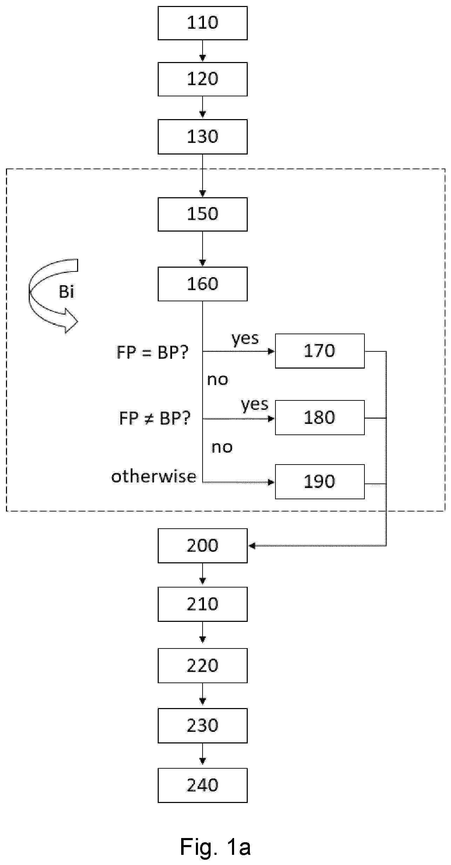

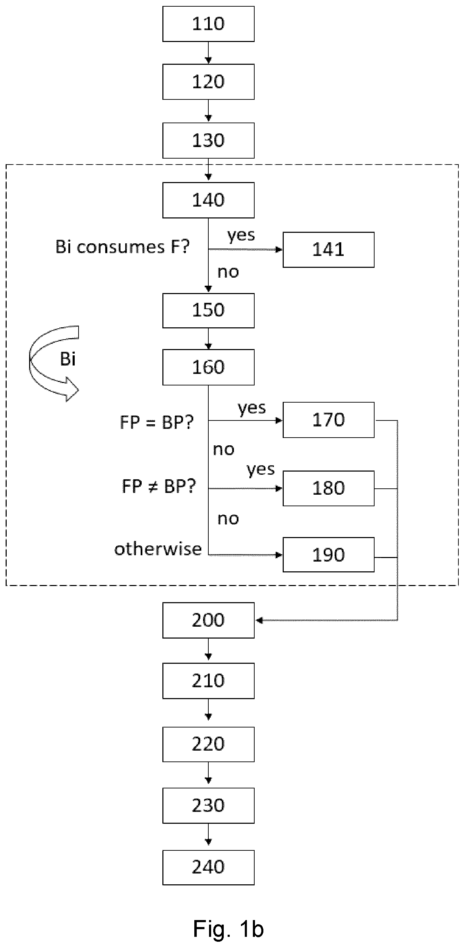

Figures 1a and 1b shows the flowcharts of two different embodiments of the method for designing a diet of the invention.

In particular, Figure 1a depicts the flowchart a computer implemented method (100) for designing a diet for a user, the method comprising the following steps:

a) accessing (110) a database (1), the database comprising

- a list of foods, each food comprising at least one food prebiotic, FP;
- a first list of bacterial species commonly found in the gut microbiome of subjects from the species to which the user belongs; and
- a list of at least one bacterium prebiotic, BP, consumed by each bacterium of the first list;

b) providing (120) a second list of bacteria comprising the bacteria lacking in the user's gut microbiome and/or the bacteria present in a low quantity in the user's gut microbiome;

b) selecting (130) a food, F; and for each bacterium, $B_i$, of the second list of bacteria; wherein i is an index from 1 to the number of bacteria of said second list:

I. obtaining (150), from the database, the list of at least one bacterium prebiotic BP consumed by the bacterium $B_i$; and

II. comparing (160) the at least one food prebiotic FP with the list of at least one bacterium prebiotic BP, such that:

i. if the at least one food prebiotic FP totally matches with the at least one bacterium prebiotic BP of the list, assigning (170) the food F with a maximum value predefined; or

ii. if none of the at least one food prebiotic FP matches with the at least one bacterium prebiotic BP of the list, assigning (180) the food F with a minimum value predefined; or

iii. if a subset of the at least one food prebiotic FP matches with the at least one bacterium prebiotic BP of the list, assigning (190) the food F with a value given by a partial sums of an harmonic series; wherein the number of elements of the harmonic series is equal to the number of food prebiotics of the subset;

d) generating (200) a vector of growth promoting for the food F, wherein each element of the vector is the value assigned to the food F for each bacterium $B_i$ of the second list of bacteria;
e) repeating (210) the previous steps for a plurality of foods;
f) calculating (220), for each food, the sum of the elements of its vector of growth promoting;
g) generating (230) a ranking of foods from the food with the highest sum of the elements of its vector of growth promoting to the food with the lowest sum of the elements of its vector of growth promoting;
h) designing (240) the diet so that it comprises at least one food of the first position or positions of the ranking, being the total number of foods of the diet predefined.

The method requires continued access (110) to a database with, at least, the following information based on scientific evidence:

- a list of foods, each food comprising at least one food prebiotic, FP;
- bacterial species commonly found in the gut microbiome of subjects from the species to which the user belongs; and
- a list of at least one bacterium prebiotic consumed by each bacterium of the first list.

Next, the method provides (120) a second list of bacteria comprising the bacteria lacking in the user's gut microbiome and/or the bacteria present in a low quantity in the user's gut microbiome. Preferably, the second list of bacteria is created according to a fecal genetic analysis.

Next, the method continues with the selection (130) of a food F and with an iterative process in which, for each of the i-th bacterium of the second list of the database, the following steps are carried out:

Firstly, the method requires obtaining (150), from the database, the list of at least one bacterium prebiotic BP consumed by the bacterium $B_i$ and comparing (160) said bacterium prebiotic or prebiotics, BP, with the at least one food prebiotic FP of the food F.

At this point, three options are available:

- The food prebiotic or prebiotics FP totally matches with the bacterium prebiotic or prebiotics BP. In this case, the food F is assigned (170) with a maximum value predefined; for example, a value of one.
- None of the food prebiotic or prebiotics FP matches with the bacterium prebiotic or prebiotics BP. In this case, the food F is assigned (180) with a minimum value predefined; for example, a value of zero.
- Part of the food prebiotics FP matches with the bacterium prebiotics BP. In this case, the value assigned (190) to the food F will depend on the number of matching prebiotics according to a partial sum of an harmonic series. In particular, the number of elements of the harmonic series will be equal to the number of food prebiotics that match with the bacterium prebiotics.

In particular, the number of elements of the harmonic series will be equal to the number of food prebiotics that match with the bacterium prebiotics and the partial sum of the harmonic series preferably as follows:

$$1 + \frac{1}{2} + \frac{1}{3} + \cdots$$

Optionally, as shown in Figure 1b, before analyzing which prebiotics of the food F are consumed by a specific bacterium $B_i$, the method firstly checks if there is an association between said bacterium $B_i$ and the food F that is endorsed directly by the scientific community. In such cases, the method grants (141) the maximum value predefined to said food F for this bacterium $B_i$.

To achieve this, this embodiment requires that the database further comprises a list with at least one food consumed by at least one of the bacterium of the first list of bacterial species.

In this way, the method requires a step of obtaining (140), from the database, the list of at least one food that the bacterium consumes (substep c1). After this, the method checks (substep c2) if the food F is comprised in this list of foods and, if so, the food F is given (141) the maximum value; for example, a normalized value of 1. After this, the method continues again in substep c1) if there are further bacteria of the second list to be analyzed or in step d) if all the bacteria have been analyzed for the given food F.

Otherwise (substep c3), i.e. when direct evidence is lacking, the intensity of the association between a bacterium $B_i$ and the food F is calculated through the prebiotics that F contains and $B_i$ consumes (again, according to scientific evidence). This means that, if the food F is not comprised in the list of foods consumed by the bacterium $B_i$, or if there is not a list with the foods consumed by the bacterium $B_i$ in the database, further checks are carried out by the method in order to assign a value to the food F; that is, the method continues with the step c.I).

The following example explains how the assignation of values is performed according to the embodiment shown in Figure 1b:

The information below is comprised in the database:

| First list of bacteria | List of foods consumed by each bacterium | List of prebiotics BP consumed by each bacterium |
|---|---|---|
| B1 | F1, F2, F3 | BP1, BP2, BP3 |
| B2 | F2 | BP1, BP2 |
| B3 | F3 | BP3 |

The foods to be analyzed are F1, F2 and F3.

The food prebiotics of F1 are FP1, FP2 and FP3.

The food prebiotics of F2 are FP1 and FP3.

The food prebiotic of F3 is FP3.

The second list of bacteria (bacteria that are lacking in the user's gut microbiome or that are present in a low quantity) comprises B1 and B2, so only B1 and B2 will be analyzed for the set of foods F1, F2 and F3.

For food F1, starting from B1, it can be seen from the database that said food F1 is consumed by the bacterium B1 so food F1 is assigned with a maximum value; for example, one.

Continuing with the bacterium B2, it can be seen from the database that the food F1 is not consumed by B2 so the method will check if the prebiotics of F1 (FP1, FP2 and FP3) are consumed totally or at least partially by the bacterium B2. In this case, two of the three prebiotics of food F1 is consumed by the bacterium B2 (FP1 is equivalent to BP1 and FP2 is equivalent to BP2) so the value assigned to the food F1 for bacterium B2 will be calculated according to the partial sum of a harmonic series. For example, a normalized value for the following harmonic series:

$$1 + \frac{1}{2} + \frac{1}{3} + \cdots$$

In this case, as two of three prebiotics are consumed by the bacterium B2, the value assigned to food F1 will be (1+1/2)/3=0.5.

The iterative process (both for embodiments shown in Figure 1a and Figure 1b) finishes once the food F has been assigned with a certain value for each of the bacterium of the second list. At this point, for the food F, a vector of growth promoting is generated (200) so that each element of the vector corresponds with the value assigned to the food F for each bacterium $B_i$.

Coming back to the previous example, the vector of growth promoting of the food F1 will be v1 = (1, 0.5).

Next, all the previous steps are repeated (210) for different kind of foods, resulting in a set of different vectors of growth promoting.

In this way, the vectors of growth promoting of foods F2 and F3 will be: v2 = (1 1) and v3 = (1 0).

A ranking of foods is then performed (230) according to the sum of elements of the vectors of growth promoting (220). The ranking begins with the food with the vector of growth promoting with the highest sum of elements to the food with the vector of growth promoting with the lowest sum of elements.

Finally, the method ends designing (240) the diet so that it comprises at least one food of the best position in the ranking. The diet can be designed with more than one food, all of them being placed in the top positions of the ranking.

In this example, the ranking will be: F2, F1 and F3 so the diet will comprise, at least, the food F2.

In an embodiment, the database further comprises a variable that defines, for each bacterium prebiotic BP, its capacity for promoting growth in the gut of each bacterium of the first list; and wherein the value assigned to the food F in step cII.iii) is further based on said variable.

In an embodiment, the variable is a binary variable which can have a first value which indicates that the bacterium prebiotic BP is a high growth promoter for the bacterium or a second value which indicates that the BP is a low growth promoter for the bacterium.

In an embodiment, the database further comprises the quantity in which each food prebiotic, FP, is present in each food; and wherein the value assigned to the food F in step cII.iii) is further based on said quantities in which the at least one food prebiotic FP is present in said food F.

In an embodiment, the value assigned to the food F in step cII.iii) is performed according to the following equation:

$$Value\_food(n) = \begin{cases} 0, & if\ n = 0 \\ \frac{1}{2} * max.\ value\ predefined, & if\ n = \frac{1}{2} \\ \left(\frac{1}{2} + w \sum_{i=2}^{2n} \frac{1}{i}\right) * max.\ value\ predefined, & otherwise \end{cases},$$

wherein w is a coefficient calculated as follows:

$$w = \frac{1}{2} / \left(\sum_{i=2}^{2N} \frac{1}{i}\right),$$

being *N* **the** total number of bacterium prebiotics contained in the database and $n = \frac{1}{2} * n_l + 1 * n_h$ , being $n_l$ the number of low-growth promoter prebiotics and $n_h$ is the number of high-growth promoter prebiotics, wherein

a low-growth promoter prebiotic is a food prebiotic that is present in the food F in a quantity below a first predefined threshold and/or a food prebiotic for which the variable that defines its capacity for promoting growth of the bacterium $B_i$ in the gut microbiome is below a second predefined threshold; and
a high-growth promoter prebiotic is a food prebiotic that is present in the food F in a quantity above or equal the first predefined threshold and/or a food prebiotic for which the variable that defines its capacity for promoting growth of the bacterium $B_i$ in the gut microbiome is above or equal to the second predefined threshold.

Coming back to the previous example, if food prebiotic FP1 is a high growth promoter and food prebiotic FP2 is a low growth promoter, the value assigned to food F1, in this embodiment, for the bacterium B2 will be calculated as follows.

$$value_{F1} = \left(\frac{1}{2} + w \sum_{i=2}^{2n} \frac{1}{i}\right) * max.\ value\ predefined$$

Wherein n = ½ + 1 = 1.5;
N = 3;

$$w = \frac{1}{2} / \left(\sum_{i=2}^{2N} \frac{1}{i}\right) = \frac{1}{2} / \left(\sum_{i=2}^{6} \frac{1}{i}\right) = \frac{1}{2} / 1.45 = 0.34$$

So, if the maximum value predefined is one, the value assigned to the food F1 will be:

$$value_{F1} = \left(\frac{1}{2} + 0.34 \sum_{i=2}^{3} \frac{1}{i}\right) * 1 = \left(\frac{1}{2} + 0.34 * 0.83\right) = 0.787$$

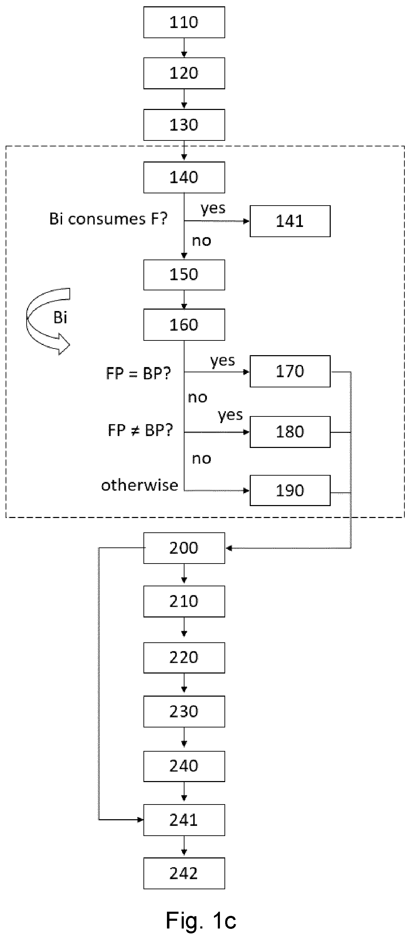

Figure 1c shows other flowchart according to an embodiment of the method of the invention. This method, as compared with the one depicted in Figure 1b, further comprises the following sub-steps:

h1) calculating (241) the Euclidean distance between the vector of growth promoting of the food or foods of the diet and each of the vectors of growth promoting of the rest of the foods, if any;
h2) designing (242) the diet so that it further comprises the foods for which the distances estimated are the biggest ones, being the total number of foods of the diet predetermined.

The sub-steps h1) and h2) can be also performed for the embodiment shown in Figure 1a.
For example, the following vectors of growth promoting belong, respectively, to three different food F4, F5 and F6 when analyzing five different target bacteria:

V4 = (1; 0; 1; 0; 1);

V5 = (1; 1; 0; 0; 0);

V6 = (0; 1; 0; 1; 0).

The food F4 is the one selected to be comprised in the diet because its sum of elements is the highest one, being therefore said food F4 on the top of the food ranking. If the diet is improved complementing F4 with either F5 or F6, the method of the invention according to this embodiment selects F6 as the best option because F4 and F6, together, will cover all five bacteria while the combination of F4 and F5 will just feed four bacteria.

This idea is behind the estimation of the Euclidean distance between the vectors, which is calculated as follows:

$$euc.\,distance_{v4-v5} = \sqrt{(x2-x1)^2 + \cdots + (z2-1)^2} = \sqrt{0^2 + 1^2 + 1^2 + 0^2 + 1^2} = 1.73$$

$$euc.\,distance_{v4-v6} = \sqrt{(x2-x1)^2 + \cdots + (z2-1)^2} = \sqrt{1^2 + 1^2 + 1^2 + 1^2 + 1^2} = 2.24$$

In this way, the combination of foods with the biggest Euclidean distance is suitable to be part of the personalized diet as they will feed a greater number of target bacteria.

In a preferred embodiment, the estimation of the Euclidean distances of sub-step h1) is performed using an agglomerative hierarchical clustering method. In a more preferred example, the agglomerative hierarchical clustering method is an Unweighted Pair Group Method with Arithmetic mean (UPGMA).

This algorithm, used in bioinformatics for the creation of phenograms, produces rooted dendrograms and requires a constant-rate assumption (that is, it assumes an ultrametric tree in which the distances from the root to every branch tip are equal). Alternative linkage schemes generate different dendrograms because of the formula used to compute the inter-cluster distance. In the case of the UPGMA, the distance between two clusters Ci and Cj involves considering the size of their subclusters:

$$D\left(C_i, C_j\right) = \frac{n_{i1}\,d\left(C_{i1}, C_j\right) + n_{i2}\,d\left(C_{i2}, C_j\right)}{n_{i1} + n_{i2}}$$

where $C_{i1}$ and $C_{i2}$ are the two clusters that join to form $C_i$ and $n_{i1}$ and $n_{i2}$ are their respective cardinalities.

An agglomerative clustering method starts by assigning each point to an individual cluster (so the number of clusters is initially equal to the number of points), and then proceeds by grouping together those clusters that are at the smallest intercluster distance. The algorithm finishes when all the points are grouped in only one big cluster. The final result can be easily interpreted by reading its corresponding dendrogram.

Foods that share the same vector representation (similar foods) are the first to be grouped together, and those that belong to groups that are farther apart in this high-dimensional space (foods that complement each other) will be among the last to be joined in a cluster. The experimental results demonstrate that picking up ingredients from distinct groups almost always leads to more complete diets.

In a particular embodiment, the foods are selected from a group consisting of Açaí, Brown algae, Corn Hazelnuts, Onion, Sarda, Agave, Brussels sprouts, Corn bran, Honey, Passion fruit, Sea buckthorn, Almonds, Buckwheat, Cucumber, Hypericum perforatum, Peanuts, Sesame, Amaranth, Capers, Curcuma longa, Integral rice, Pear Sorghum, Apples, Carrots, Dark chocolate, Jerusalem artichoke, Peppers, Sorghum bran, Apricot, Cashew nuts, Date, Kiwi, Pineapple, Soy, Artichoke, Cassava, Echinacea purpurea, Kuding tea, Pistachio, Spelt, Asparagus, Cauliflower, Egg, Larch Plantains, Spinach, Avocado, Celery, Eggplant, Leek, Plums, Spirulina alga, Bamboo shoots, Cheese, Endives, Lentils, Pomegranate, Strawberries, Bananas, Cherries, Feijoa, Longan, Potato, Sugar cane, Barley, Chestnuts, Fermented soy milk, Lotus seeds, Pumpkin, Sunflower, Barley bran, Chia seeds, Figs, Mango, Quince, Beer, Chicken, Garlic, Milk, Quinoa, Sweet potato, Beer yeast, Chickpeas, Ginkgo biloba, Millet, Radishes, Tomato, Beetroot, Chicory, Goji, Berries, Millet bran, Raspberries, Tuna, Black tea, Citrus, Gooseberries, Mushrooms-fungi, Red wine, Turkey, Blackberries, Cocoa powder, Grapes, Oat bran, Rice bran, Walnuts, Blueberries, Coconut, Green Tea, Oats, Rye, Watercress, Broad beans, Codonopsis pilosula, Green beans, Olive oil, Rye bran, Wheat, Broccoli, Coffee, Green peas, Olives, Salmon, Wheat bran, White lupin, Yacon, Yam and any combination thereof.

In a particular embodiment, the food prebiotics and the bacterium prebiotics are selected from a group consisting of xylooligosaccharides (XOS), resistant starch, fructooligosaccharides (FOS), inulin, pectin, polyphenols, resveratrol, quercetin, raffinose, arabinoxylan (AX), arabinoxylan oligosaccharides (AXOS), arabinogalactans, galacto-oligosaccharides (GOS), beta-glucans, tryptophan, mannan-oligosaccharides (MOS), lignans, ellagitannins, curcumin, anthocyanins, isoflavones, and any combination thereof.

In a particular embodiment, the bacteria of the database are selected from a group consisting of *Bacillus, Eubacterium rectale, Barnesiella, Faecalibacterium prausnitzii, Bifidobacterium, Lactobacillus acidophilus, Butyrivibrio, Lactobacillus brevis, Lachnospira, Lactobacillus delbrueckii, Leuconostoc, Lactobacillus casei, Oscillospira, Lactobacillus fermentum, Weisella, Lactobacillus paracasei, Bacillus subtilis, Lactobacillus plantarum, Bifidobacterium adoles-*

*centis, Lactobacillus reuteri, Bifidobacterium animalis, Lactobacillus rhamnosus, Bifidobacterium bifidum, Lactobacillus salivarius, Bifidobacterium longum, Leuconostoc mesenteroides, Collinsella aerofaciens, Roseburia hominis, Coprococcus catus, Roseburia inulinivorans, Enterococcus faecium, Selenomonas ruminantium, Eubacterium hallii, Streptococcus thermophiles, Anaerostipes, Anaerotruncus, Bacteroides, Butyricimonas, Gemmiger, Haemophilus, Lactococcus, Oscillibacter, Parabacteroides, Phascolarctobacterium, Subdoligranulum, Akkermansia muciniphila, Anaerostipes hadrus, Lactobacillus, Roseburia, Anaerotruncus colihominis, Bacteroides uniformis, Bacteroides vulgatus, Christensenella minuta, Clostridium butyricum, Clostridium leptum, Coprococcus, Coprococcus eutactus, Lactococcus lactis, Oxolobacter formigenes, Roseburia faecis, Roseburia intestinalis, Roseburia inulinivorans,* and any combination thereof.

Data processing system, computer program and computer-readable medium

In another aspect, the invention relates to a data processing system comprising means for carrying out the steps of the method of the invention.

In another aspect, the invention relates to a computer program comprising instructions that, when the program is executed by a computer, cause the computer to carry out the steps of the method of the invention.

The term computer must be interpreted in a non-restrictive manner, not only as a personal computer but rather as a set of computational resources capable of carrying out the instructions constituting the computer program. This is the case, for example, of execution modes identified "as a service", such as execution platforms in the cloud, for example, where execution is done remotely. This is also the case of mobile terminals referred to as smartphones, which can be identified as computers capable of executing programs, normally referred to as "Apps".

In another aspect, the invention relates to a computer-readable medium comprising instructions that, when executed by a computer, cause the computer to carry out the steps of the method of the invention.

All the terms and embodiments previously described in relation to the combination of the invention are equally applicable to these aspects of the invention.

Method for increasing the richness of the gut microbiome in a user and method for increasing the content of a prebiotic within the gut microbiome in a user

It is also described an unclaimed method for increasing the richness of the gut microbiome in a user comprising

(i) selecting a diet for the user by the method according to the first inventive aspect and
(ii) administering the diet selected in step (i) to the user for a sufficient amount of time to effect a change in the gut microbiome.

It is also described an unclaimed method for increasing the content of a probiotic within the gut microbiome in a user comprising

(i) selecting a diet for the user by the method according to the first inventive aspect, wherein the second list of bacteria provided in step b) consist of a single bacterium which coincides with probiotic whose contents are to be increased and
(ii) administering the diet selected in step (i) to the user for a sufficient amount of time to effect a change in the gut microbiome.

"Richness" in relation to the gut microbiome, as used herein, relates to the total number of bacterial species in the gut microbiome.

"Increasing" in relation to the richness or content of a probiotic, as used herein indicates that the level of a single bacterium after administering the selected diet is higher compared to the level of said bacterium before administering the selected diet to the subject. The level is considered increased when it is at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more higher than the level of said bacterium in the gut microbiome before administering the selected diet.

The level of a particular bacterium can be quantified by any method known in the art, for example by counting the total number of colony-forming units (CFUs) grown on an agar plate from serial dilutions, expressed as CFU per gram or mL of the original sample. This yields an estimate of the number of cells present based on a skilled interpretation of the number of colonies on a plate. In addition, the quantification can be performed, for example, by real-time PCR or flow cytometry.

In a preferred embodiment, the bacterium is selected from the group consisting of *Bacillus, Eubacterium rectale, Barnesiella, Faecalibacterium prausnitzii, Bifidobacterium, Lactobacillus acidophilus, Butyrivibrio, Lactobacillus brevis,*

*Lachnospira, Lactobacillus delbrueckii, Leuconostoc, Lactobacillus casei, Oscillospira, Lactobacillus fermentum, Weisella, Lactobacillus paracasei, Bacillus subtilis, Lactobacillus plantarum, Bifidobacterium adolescentis, Lactobacillus reuteri, Bifidobacterium animalis, Lactobacillus rhamnosus, Bifidobacterium bifidum, Lactobacillus salivarius, Bifidobacterium longum, Leuconostoc mesenteroides, Collinsella aerofaciens, Roseburia hominis, Coprococcus catus, Roseburia inulinivorans, Enterococcus faecium, Selenomonas ruminantium, Eubacterium hallii, Streptococcus thermophiles, Anaerostipes, Anaerotruncus, Bacteroides, Butyricimonas, Gemmiger, Haemophilus, Lactococcus, Oscillibacter, Parabacteroides, Phascolarctobacterium, Subdoligranulum, Akkermansia muciniphila, Anaerostipes hadrus, Lactobacillus, Roseburia, Anaerotruncus colihominis, Bacteroides uniformis, Bacteroides vulgatus, Christensenella minuta, Clostridium butyricum, Clostridium leptum, Coprococcus, Coprococcus eutactus, Lactococcus lactis, Oxolobacter formigenes, Roseburia faecis, Roseburia intestinalis, Roseburia inulinivorans,*

"Sufficient amount of time", as used herein relates to the sufficient amount of time to provide the desired effect and it will generally be determined by, among other causes, the characteristics of the diet itself and the effect to be achieved. The time for obtaining a change in the gut microbiome can also depend on a range of factors, such as, for example, age, weight, sex or health condition of the user. According to the invention, the desired effect is a change in the gut microbiome.

"A change in the gut microbiome", as used herein, means that the number of probiotics or a particular probiotic is increased in the gut microbiome.

All the terms and embodiments previously described in relation to the combination of the invention are equally applicable to these aspects of the invention.

## *Examples*

The following examples illustrate the invention and must not be considered as limiting the same.

### *Material and methods*

1. Literature review

The inventors have carried out a systematic review to choose the most significant bacteria at genus and species level advances in association with prebiotics, and food components in the scientific literature listed in Pubmed and Pubmed Central Repositories of NCBI (National Center of Biotechnology Institute), after searching for "gut microbiome" AND ("prebiotic" OR "probiotic"), published until March of 2021.

2. Case studies

Two case studies were carried out. The first one consists of gut microbiome analysis and the food recommender method (in particular, in form of an application) in a healthy volunteer, a European Caucasoid origin 39 years old female ("A food recommender system for a better gut microbiome balance." Article waiting for press).

The second case study consists of gut microbiome analysis pre-and post- food recommender method (in particular, in form of an application) in a 12 weeks dietary intervention. In this case, the participant followed a western diet the 9 weeks before the intervention. The dietary intervention based on the present food recommender method lasted 12 weeks and was supervised by a nutrition professional. ("Second case study: precision nutrition-based practical dietary intervention." Included in the present file).

2.1. Personal data protection and ethical approval

They were adequately informed about risk and benefits, according to the EU General Data Protection Regulation (GDPR), the Regulation (EU) 2016/679 of the European Parliament and of the Council of 27 April 2016 on the protection of natural persons about the processing of personal data and on the free movement of such data, and repealing Directive 95/46/EC (General Data Protection Regulation), OJ 2016 L 119/1. The present study was approved by the Human Research Ethics Committee of the School of Science and Technology, University of the Basque Country (UPV/EHU) (M10_2018_039).

2.2. Molecular analysis

2.2.1. Sampling

Feces samples were taken between 7-8 AM prior and after 12 weeks of nutritional intervention by the DANASTOOL Sample Collection Microbiome Kit of DANAGEN® (Barcelona, Spain) following the manufacturer's indications. Although microbiome composition keeps stable in the collection kit for at least one month (data not shown), samples were stored at -80°C until DNA extraction was carried out.

2.2.2. DNA extraction and quantitation

DNA was extracted using the DANAGENE Microbiome Fecal DNA kit (Barcelona, Spain). DNA Quantitation was carried out by fluorometry (Qubit 2.0, Life Technologies, ThermoFisher Scientific, USA) using HS dsDNA Assay (Thermo-Fisher Scientific, USA) and by spectrophotometry (NanoDrop 2000c, ThermoFisher Scientific, USA).

2.2.3. 16S rRNA gen amplification by PCR

PCR amplification of full-length 16S rRNA genes (1500 pb) was conducted using the 16S Barcoding Kit (SQK-RAB204; Oxford Nanopore Technologies, Oxford, UK) containing the 27F/1492R primer set and LongAmp™ Taq 2× Master Mix (New England Biolabs, Ipswich, MA, USA). Amplification was performed using an Applied Biosystems Veriti™ Thermal Cycler (Thermo Fischer Scientific, Waltham, MA, USA) with the following PCR conditions indicated by the manufacturer: initial denaturation at 95 °C for 3 min, 25 cycles of 95 °C for 20 s, 55 °C for 30 s, and 65 °C for 2 min, followed by a final extension at 65 °C for 5 min (Kai, S. et al. Rapid bacterial identification by direct PCR amplification of 16S rRNA genes using the MinION™ nanopore sequencer. FEBS Open Bio 9, 548-557 (2019)).

2.2.4. Library preparation and sequencing of 16S rRNA gene amplicons

PCR products were purified using Clean NGS (CleanNA, PH Waddinxveen, The Netherlands) and quantified by fluorometry (Qubit 4.0, Life Technologies, ThermoFisher Scientific, USA) using HS dsDNA Assay (ThermoFisher Scientific, USA). A total of 100 ng DNA was used for library preparation, and MinION™ sequencing was performed using MinION nanopore DNA sequencer, ADP-FLG001 Flonge adapter, and FLO-FLG001 (Oxford Nanopore Technologies, United Kingdom) according to the manufacturer's instructions.

2.3. Data acquisition and Bioinformatic processing

minknow UI software ver. 21.02.01 (Oxford Nanopore Technologies, United Kingdom) was used for data acquisition and base-calling converting sequence reads (i.e., FAST5 data) were into FASTQ files by Guppy version 3.2.10 pipeline (Oxford Nanopore Technologies, United Kingdom). The Barcoding workflow in the Metrichor Ltd. analysis platform EPI2ME (Oxford Nanopore Technologies, United Kingdom) was used to identify bacteria at the genus and species level. For that purpose, fastq files were uploaded to the EPI2ME desktop agent 16S workflow (Oxford Nanopore Technologies, United Kingdom), where real-time classification was carried out using the NCBI 16S rRNA gene blast database. The blast was run using the parameters max_target seqs = 3 (finds the top three statistically significant hits)- All read classifications were filtered for >77% accuracy and >30% coverage, removing non-specific alignments. Obtained results were downloaded as a comma-separated values (CSV) file. In-house software results were processed to avoid infra representation of each taxonomical ID and convert reads in relative abundance according to estimated 16S copy number for each bacterium, based on the rrnDN database (Stoddard, S. F., Smith, B. J., Hein, R., Roller, B. R. K. & Schmidt, T. M. rrnDB: improved tools for interpreting rRNA gene abundance in bacteria and archaea and a new foundation for future development. Nucleic Acids Res 43, D593-D598 (2015)).

2.4. Control DNA study for sensitivity and specificity determination

ZymoBIOMICS gut Microbiome Standard (Zymo Research, Irvine, CA) was used as Control DNA and analysed twice, following the same lab methodology of the case study to determine the sensitivity and specificity of the molecular analysis.

3. Analysis pre-and post-intervention

The richness of the gut microbiome of the pre-and post- nutritional intervention samples was measured by counting the number of different species and calculating the Margalef Index (R)[3]:

$$R = \frac{S-1}{ln(n)} \quad \text{where} \quad \left\{ \begin{array}{l} S = \text{number of species} \\ n = \text{number of observed individuals} \end{array} \right.$$

The number of individuals and the relative abundance of the species was also measured.

*Example 1- Methodology*

First, the method estimates the contribution of food ingredients to improving each bacterium on a scale of 0 to 1 (i.e., no ingredient or group of ingredients can have a bigger than one influence on a certain bacteria).
To do so, the method relies on two indicators:

- when the association is endorsed directly by the scientific community, the method grants the maximum value to the given pair of (food, bacterium).
- when direct evidence is lacking, the intensity of the association between a bacterium B and a food ingredient F is calculated through the prebiotics that F contains and B consumes (again, according to scientific evidence).

More specifically:

- if F and B contain all the prebiotics used in the study, the value assigned to the pair (F, B) is 1. The choice is based on the following main reasons:

  1) the scientific evidence regarding associations between microbiome and nutrition is growing exponentially in the last years, continuously discovering new connections between specific bacteria and prebiotic components; thus, aiming to minimize the error of not yet discovered interactions, the algorithm targets all prebiotics included in the study and not only those that are currently associated with the bacteria.
  2) it is useful that the values of this function to be comparable across the whole spectrum of bacteria (i.e., a food that shares the same number of prebiotics with two different bacteria should have the same value).

- If F does not contain any of the prebiotics that B consumes, the corresponding value is zero.

- In all other cases, the value grows logarithmically with the number of prebiotics that B consumes and F contains. That is, its values correspond to the partial sums of the harmonic series:

$$1 + \frac{1}{2} + \frac{1}{3} + \cdots$$

As mentioned before, according to the current scientific evidence regarding the quantity of prebiotics in each food component and the effect in the gut bacterial growth, the prebiotics are classified in each food as either "high growth promoters" or "low growth promoters" to adjust the formula to the real interactions in gut microbiome.
The function that quantifies the influence of a given food (based on its number and type of prebiotics) on the growth of specific bacteria has the following form:

$$F(n) = \left\{ \begin{array}{ll} 0, & if\ n = 0 \\ \frac{1}{2}, & if\ n = \frac{1}{2} \\ \frac{1}{2} + w \sum_{i=2}^{2n} \frac{1}{i}, & otherwise \end{array} \right.$$

where N is the total number of prebiotics considered in the study,

$$w = \frac{1}{2} / \left( \sum_{i=2}^{2N} \frac{1}{i} \right)$$

is a coefficient that was calculated to ensure F(N) = 1, n = 1/2 corresponds to one low growth promoter and n = 1 to a high growth one. Thus, the following progression is obtained:

$$
\begin{aligned}
F(0.5) &= 0.5 \\
F(1) &= F(0.5) + \frac{w}{2} \\
F(1.5) &= F(1) + \frac{w}{3} \\
&\ldots \\
F(N) &= F(N-1) + \frac{w}{2N}
\end{aligned}
$$

That is, with one prebiotic that is a low growth promoter, the expected value quantifying the influence of that nutrient or food on a given target microorganism is F(0.5) = 0.5. With two prebiotics of this kind (or equivalently, one high growth promoter), the value is F(1), with one "low" and one "high", a value of F(1.5) was obtained and so on. The peak, F(N) = 1, is reached when the food contains N prebiotics that are high growth promoters for that bacterium.

Then, each food $F_i$ is represented by an Nb-dimensional vector, where B1; B2; ...; BNb are the microorganisms under study. The value $F(n_{ij})$ of the j-th component of this vector is computed using $n_{ij}$, the size of the set of prebiotics that $F_i$ and $B_j$ have in common. Note that a prebiotic that is a high growth promoter for F adds 1 to this size, whereas a low growth promoter only 0.5.

This calculus is not limited to a single food. In order to evaluate the contribution of a set of foods, the method considers the union of those prebiotics that each individual food contains and bacterium B consumes, taking always the highest values of the association intensity for a given prebiotic (if the prebiotic is a high growth promoter in at least one food, it will count as a high growth promoter; otherwise, its contribution will be that of a low growth promoter).

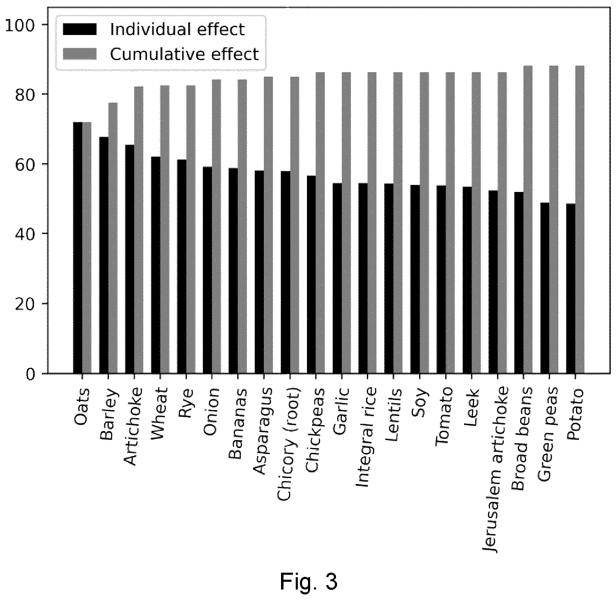

Once the inventors have quantified the extent to which each (set of) food(s) contributes to improving a certain bacterium that a user's microbiome lacks (or only has in a small quantity), the method calculates the total contribution of each (set of) food(s) to the user's microbiome by simply adding together all these amounts. Food ranking will be determined by this total value: the higher the value, the better the food in the sense that it covers a wider range of bacteria. Thus, both the individual effect and the cumulative total effect of the top k food ingredients can be visualized (an example is given in Figure 3).

Going back to the original problem that this approach tackles: What does a user eat to get the best outcome? The method is a way to assess the contribution of a certain dish to the user's general health based on its ingredients. Moreover, this procedure can be applied for any set of target bacteria, so the method can specialize the search using as target only those that have the impact being pursuit (weight control, emotional balance, intestinal health, increased metabolism rate, energy intake, etc.).

But how do we choose which foods should be combined together in this perfect dish in the first place? As it has been mentioned before, this is a computationally expensive problem to solve. So instead of searching for the BEST dish, the method proposes to settle for those dishes that feed as many bacteria as possible by making use of known data mining techniques. This allows for diversity in the user's daily food intake without compromising too much on the effects it has on his or her microbiome.

Thus, having as inputs the vectors corresponding to each food, the method employs an agglomerative (bottom-up) hierarchical clustering method called UPGMA (unweighted pair group method with arithmetic mean).

Another advantage of using this method is its time complexity. A trivial implementation of the algorithm to construct the UPGMA tree has $O(n^3)$ time complexity, and using a heap for each cluster to keep its distances from other clusters reduces its time to $O(n^2 \log n)$.

*Example 2- First Case study*

The food recommender method was tested on a real case study. A set of 104 target bacteria was selected, out which 34 (listed in Table II) were found to be either missing or in insufficient quantities in the test bed.

Table II

| Target bacteria | |
|---|---|
| Bacillus | Eubacterium rectale |
| Barnesiella | Faecalibacterium prausnitzii |
| Bifidobacterium | Lactobacillus acidophilus |
| Butyrivibrio | Lactobacillus brevis |
| Lachnospira | Lactobacillus delbrueckii |

(continued)

| Target bacteria | |
|---|---|
| Leuconostoc | Lactobacillus casei |
| Oscillospira | Lactobacillus fermentum |
| Weisella | Lactobacillus paracasei |
| Bacillus subtilis | Lactobacillus plantarum |
| Bifidobacterium adolescentis | Lactobacillus reuteri |
| Bifidobacterium animalis | Lactobacillus rhamnosus |
| Bifidobacterium bifidum | Lactobacillus salivarius |
| Bifidobacterium longum | Leuconostoc mesenteroides |
| Collinsella aerofaciens | Roseburia hominis |
| Coprococcus catus | Roseburia inulinivorans |
| Enterococcus faecium | Selenomonas ruminantium |
| Eubacterium hallii | Streptococcus thermophilus |

The methodology implements progressively new advances in the association between gut microbiome, prebiotics, probiotics and food components. In this study, the 16 most represented prebiotic components in the scientific literature listed in Pubmed and Pubmed Central Repositories of NCBI (National Center of Biotechonology Institute) were chosen, after searching for "gut microbiome" AND ("prebiotic" OR "probiotic"), published until August of 2020.

The set of prebiotics considered included the following species: xylooligosaccharides (xos), resistant starch, fructooligosaccharides (fos), inulin, pectin, polyphenols, resveratrol, quercetin, raffinose, arabinoxylan (ax), arabinoxylanoligosaccharides (axos), arabinogalactans, galacto-oligosaccharides (gos), beta-glucans, tryptophan and mannan-oligosaccharides (mos).

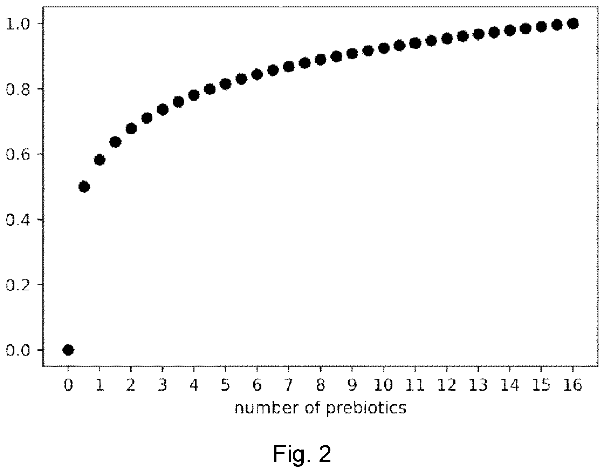

As it can be appreciated from the graph of the function (Figure 2), the maximum value of F is reached when a (group of) food ingredient(s) contains all 16 prebiotics as high growth promoters.

More than one hundred ingredients or foods were considered in the study (see Table III), and the top three most complete foods for this target set of bacteria turned up to be the oat (with a total contribution of 24.47 points, thus covering 71.97% of the whole set), the barley and the artichoke.

Table III

| The set of foods considered in the study | | | | | |
|---|---|---|---|---|---|
| | Brown algae | Corn | Hazelnuts | Onion | Sarda |
| Açaí | Brussels sprouts | Corn bran | | | |
| Agave Almonds | Buckwheat | Cucumber | Honey | Passion fruit | Sea buckthorn |
| Amaranth | Capers Carrots | Curcuma longa (turmeric) | Hypericum per-foratum | Peanuts Pear | Sesame Sorghum |
| Apples | Cashew nuts | Dark chocolate | Integral rice | Peppers | Sorghum bran |
| Apricot | Cassava | Date | Jerusalem artichoke | Pineapple | Soy |
| Artichoke | Cauliflower | Echinacea purpurea | Kiwi | Pistachio | Spelt |
| Asparagus | Celery | Egg (yolk) | Kuding tea | Plantains | Spinach |
| Avocado | Cheese | Eggplant | Larch | Plums | Spirulina alga (blue) |
| Bamboo shoots | Cherries Chestnuts | Endives Feijoa (Acca sellowiana) | Leek Lentils | Pomegranate Potato | Strawberries Sugar cane |
| Bananas | Chia seeds | | Longan | Pumpkin | Sunflower |
| Barley bran | Chicken | Fermented soy milk | Lotus seeds | Quince | Sweet potato (batata) |
| Barley | Chickpeas | Figs | Mango | Quinoa | Sweet potato (boniato) |
| Beer | Chicory (root) | Garlic | Milk | Radishes | Tomato |

(continued)

| The set of foods considered in the study | | | | | |
|---|---|---|---|---|---|
| Beer yeast | Citrus (peel and p ulp) | Ginkgo biloba | Millet | Raspberries | Tuna |
| Beetroot | | Goji Berries | Millet bran | Red wine | Turkey |
| Black tea | Cocoa powder | Gooseberries | Mushrooms-fungi | Rice bran | Walnuts |
| Blackberries | Coconut | Grapes | Oat bran | Rye | Watercress |
| Blueberries | Codonopsis | Green Tea | Oats | Rye bran | Wheat |
| Broad beans | pilosula | Green beans | Olive oil | Salmon | Wheat bran |
| Broccoli | Coffee | Green peas | Olives | | |
| White lupin | Yacon | Yam | | | |

In Figure 3 the first 20 nutrients in the ranking are listed (in decreasing order of importance) with their individual effect, along with the cumulative effect of the top k elements (with k in the range 1; 2; : : : ; 20).

Let us now consider the 34-dimension vector of the artichoke (here only its first and last elements are listed, for illustration purposes): (0.00; 0.58; 1.00; 0.71; 0.50; 0.00;... ; 0.71). Its forth component (corresponding to *Butyrivibrio*) has value 0.71 since among the five prebiotics that help it grow (XOS, RESISTANT STARCH, FOS, INULIN, PECTIN), the artichoke contains one low-growth (PECTIN) and two high-growth promoters (FOS, INULIN).

Thus, this value is given by

$$F(2 * 1 + 1 * 0.5 == F(2.5)$$

$$\frac{1}{2} + w * \left( \frac{1}{2} + \frac{1}{3} + \frac{1}{4} + \frac{1}{5} \right)$$

The value 1 assigned to the third component of the vector (Bifidobacterium) is based on Z. Zeaiter, M. E. et al., BioMed research international, vol. 2019 that finds a direct association between this bacterium and the artichoke.

The sixth one *(Leuconostoc)* has value 0 because the only prebiotic that makes it grow, the RAFFINOSE, is not present in artichokes. Therefore, any nutrient that contains this prebiotic (such as broad beans) would be a very good way of complementing its influence on the microbiome. And this is reflected by the numbers: the cumulative effect of artichoke and broad beans is 75.72%, increasing by more than 10% their individual contribution (artichoke alone 65.39%, broad beans alone 51.93%).

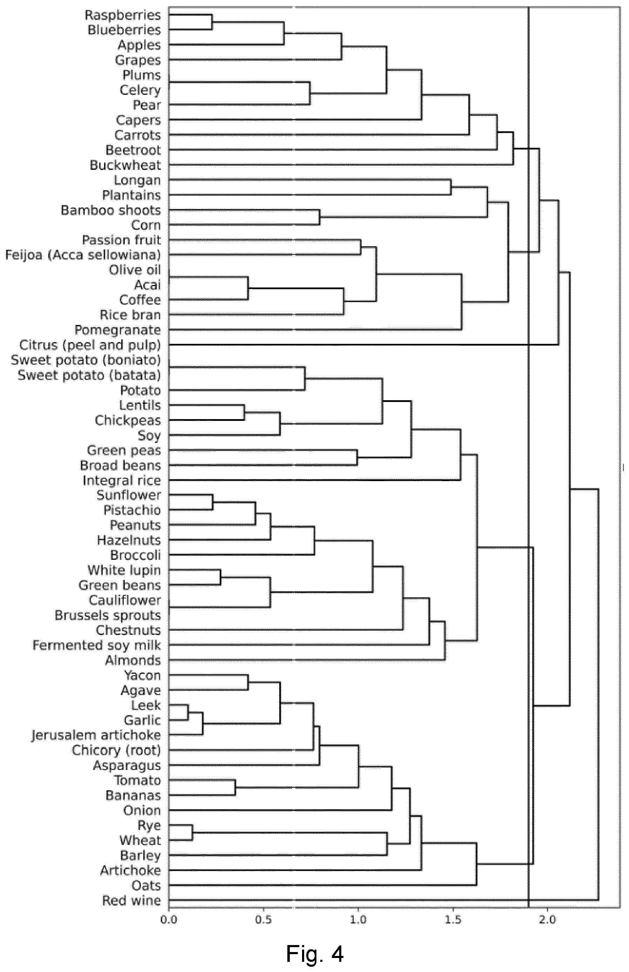

Now, let us see how these ingredients can be combined to get the best outcome. The UPGMA algorithm is applied to the top 60 highly ranked foods (roughly half of all the food types investigated) and obtained the dendrogram depicted in Figure 4. These first 60 elements already cover 93.09% of the target set, whereas using all 129 only warrants a 94.63% coverage (that is, a mere 1.54 percentage point increase).

Depending on where the line is drawn, this representation allows to distinguish as many groups as wanted. In this example, six clusters can be differentiated with an inter-cluster distance of at most 1.9. Note that one of the groups only contains one element (the red wine) and those elements that are "identical" can be clearly distinguished based on their contribution to this target set (for example, Sweet potato (batata) and Sweet potato (boniato), for which there is actually only one English term and Brussels sprouts with Cauliflower). Some less obvious examples are Acai with Olive oil or Celery with Plums. Eating a dish that contains one ingredient from each of the six groups maximizes the effect of our food intake to our microbiome. In order to test this hypothesis, the inventors devised the following two experiments. In the first one, six out of the 60 possible foods were randomly selected and the total cumulative effect of these six foods was computed. It was repeated 10; 100; 1000; 10000 and 100000 times, respectively, and the average total cumulative effect and the time it took to perform each of those tests were reported. In the second experiment, one element from each of the six available groups were selected and the same number of repetitions as in the first experiment was performed.

It can be seen that, on average, the total cumulative effect of the "guided" selection (the one that takes into consideration the groups) sees a 3-percentage point increase or more in all the five tests performed. Therefore, it is safe to say that basing a diet on dishes that combine food ingredients from different categories is more beneficial to the health.

Note that determining the "perfect six-ingredients dish" (the one that obtains maximum benefits with exactly six ingredients) is computationally challenging since it has to analyze 50 063 860 possibilities (recall that we limit ourselves to the best 60 foods). On the other hand, obtaining the best "guided" selection (110 250 possibilities) took 74.46 seconds.

This one covers already 86.78% of the target set, only 2 percent points less than the optimal one, which covers 89.04% and took 8 hours and 26 minutes to run. Not to mention that even if it is not found which is that perfect combination, eating the same thing every day is not reasonable. Instead, the recommender method and system provide an easy way to choose from a wide variety of good enough combinations.

An important observation is that this analysis can be done for any subgroup of foods (for example, including only those that a person regularly eats).

Thus, the mid-term effects of basing a diet on the guidance provided by the present methodology are promising. In a case study, after a three months period of mainly consuming food chosen by our system, state of microbiome composition was significantly improved regarding the specific dysbiosis described for phenotypes such as depression and anti-inflammatory compound production (butyrate and propionate).

These kind of strategies present the advantage of being based on more accessible resources such as food component selection and lifestyle habits, in comparison with methodologies based on surgeries and drugs. All of those can be especially essential features in a COVID-19 pandemic context in which clinical resources, and in particular those face-to-face, are limited. In addition, it implies a paradigm shift in the relationship between patient and medicine. It promotes the change from a relatively passive or expectant attitude to an active, auto-responsible one. From the position of waiting for a donor or a surgery to a proactive approach based on carrying out precision nutrition and on acquiring healthy life habits to improve microbiome balance.

*Example 2- PRECISION NUTRITION BASED PRACTICAL DIETARY INTERVENTION*

The food recommender method was tested on a second real case study. In this case, a set of 37 target bacteria was selected, out of which 24 (listed in Table IV) were found to be missing in our tested.

| NCBI Taxid | Taxid | Taxonomic level | Pre- Relative abundance (%) | Post- Relative abundance (%) | INTESTINAL PERMEABILITY | INFLAMMATORY BOWEL DISEASE | OBESITY | TYPE II DIABETES | RHEUMATOID ARTHRITIS | HYPERCHOLESTEROLEMIA | KIDNEY STONES | PROBIOTICS | BUTYRATE PRODUCTION | PROPIONATE PRODUCTION | VITAMINS | NEUROTRANSMITTERS |
|---|---|---|---|---|---|---|---|---|---|---|---|---|---|---|---|---|
| 207244 | *Anaerostipes* | Genera | 0.10 | 0.09 | | ■ | | ■ | | | ■ | | ■ | | | |
| 244127 | *Anaerotruncus* | | 0 | 0.14 | | | | ■ | | | | | ■ | | | |
| 816 | *Bacteroides* | | 1.02 | 0.61 | | ■ | ■ | ■ | ■ | ■ | * | | | | ■ | ■ |
| 397864 | *Barnesiella* | | 0.05 | 0.01 | ■ | | | | | | | | | | ■ | |
| 574697 | *Butyricimonas* | | 0.03 | 0.01 | ■ | ■ | | | | ■ | ■ | | | | | |
| 33042 | *Coprococcus* | | 0 | 1.30 | ■ | ■ | | ■ | | | | | ■ | | | |
| 1730 | *Eubacterium* | | 0 | 0.89 | ■ | | | ■ | | ■ | ■ | | ■ | | | |
| 216851 | *Faecalibacterium* | | 0 | 59.39 | ■ | ■ | ■ | ■ | ■ | ■ | | | | | | |
| 204475 | *Gemmiger* | | 0 | 4.62 | | | | | | | | | | | | |
| 724 | *Haemophilus* | | 0.36 | 0.04 | | | | | ■ | | | | | | ■ | |
| 28050 | *Lachnospira* | | 0 | 0.37 | ■ | ■ | | ■ | | | | | | | | |
| 1578 | *Lactobacillus* | | 0 | 0.56 | ■ | | | | | ■ | ■ | ■ | ■ | | ■ | ■ |
| 1357 | *Lactococcus* | | 0 | 0.01 | | | | | | | | ■ | | | | |
| 459786 | *Oscillibacter* | | 2.17 | 3.19 | | ■ | | ■ | | | | | ■ | | | |
| 375288 | *Parabacteroides* | | 0.17 | 0.07 | | ■ | | | | ■ | | | | | ■ | ■ |
| 33024 | *Phascolarctobacterium* | | 0 | 0.01 | ■ | | | | | | | | | ■ | | |
| 841 | *Roseburia* | | 3.96 | 1.38 | | ■ | | ■ | | ■ | | | ■ | | | |
| 292632 | *Subdoligranulum* | | 0 | 0.07 | | | | | | | | | | | | |
| 239935 | *Akkermansia muciniphila* | Species | 0 | 0.05 | ■ | | ■ | ■ | | ■ | | ■ | | ■ | ■ | |
| 649756 | *Anaerostipes hadrus* | | 0.10 | 0.09 | | | | | | | | | ■ | | | |
| 169435 | *Anaerotruncus colihominis* | | 0 | 0.04 | | | ■ | | | | | | | | | |
| 820 | *Bacteroides uniformis* | | 0.18 | 0.9 | | | | | | | | | | | ■ | |
| 821 | *Bacteroides vulgatus* | | 0.33 | 0.08 | | ■ | | | | | | | | | ■ | |
| 626937 | *Christensenella minuta* | | 0.03 | 0.18 | | ■ | | | | | | | | | | |
| 1492 | *Clostridium butyricum* | | 0 | 0 | ■ | | | | | | | | ■ | | | |
| 1535 | *Clostridium leptum* | | 0 | 0.06 | | ■ | | ■ | | | | | | | | |
| 116085 | *Coprococcus catus* | | 0 | 0.07 | | | | | | | | | ■ | ■ | | |
| 33043 | *Coprococcus eutactus* | | 0 | 1.13 | | | | | | | | | ■ | | | |
| 39488 | *Eubacterium hallii* | | 0 | 0.07 | ■ | | | | | | | | ■ | | | |
| 39491 | *Eubacterium rectale* | | 0 | 0.91 | ■ | ■ | | | | | | | ■ | | | |
| 853 | *Faecalibacterium prausnitzii* | | 0 | 59.39 | ■ | ■ | ■ | ■ | ■ | ■ | | | | | | |
| 1358 | *Lactococcus lactis* | | 0 | 0 | | | | | | | | ■ | | | ■ | ■ |
| 847 | *Oxalobacter formigenes* | | 0 | 0.02 | | | | | | ■ | | | | | | |
| 301302 | *Roseburia faecis* | | 0.83 | 0.61 | | | | | | | | | ■ | | | |
| 301301 | *Roseburia hominis* | | 0.87 | 0.32 | ■ | | | | | | | | ■ | | | |
| 166486 | *Roseburia intestinalis* | | 1.54 | 0.19 | ■ | ■ | | ■ | | | | | ■ | | | |
| 360807 | *Roseburia inulinivorans* | | 0.73 | 0.26 | | ■ | | ■ | | | | | ■ | | | |

Table IV:. Based on the present food recommender method, relative abundance (%) of target bacteria prior and after dietary intervention. Black boxes indicate phenotypes in which each taxon's enhanced relative abundance has been associated with positive/protective effects. For example, the asterisk indicates a significantly higher relative abundance of Bacteroides has been previously reported in patients with kidney stones disease vs. control group (34.9 vs. 10.2) (Jm, S. *et al.* Evidence for a distinct gut microbiome in kidney stone formers compared to non-stone formers. *Urolithiasis* 44, (2016).)

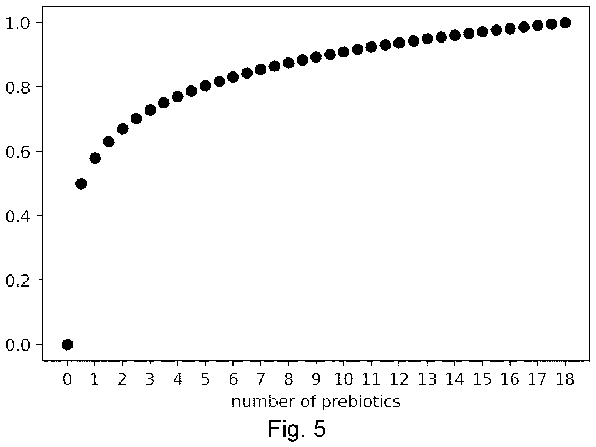

The present methodology implements progressively new advances in the association between the gut microbiome, prebiotics, probiotics, and food components. The results shown herein has chosen the 18 most represented prebiotic components in the scientific literature listed in Pubmed and Pubmed Central Repositories of NCBI (National Center of Biotechnology Institute). The set of prebiotics considered included the following species: xylooligosaccharides (XOS), resistant starch, fructooligosaccharides (FOS), inulin, pectin, resveratrol, quercetin, raffinose, arabinoxylan (AX), arabinoxylan oligosaccharides (AXOS), arabinogalactans, galacto-oligosaccharides (GOS), beta-glucans, lignans, ellagitannins, curcumin, anthocyanins, and isoflavones. As it can be appreciated from the graph of the function (Figure 5), the maximum value of F is reached when a (group of) food ingredient(s) contains all 18 prebiotics as high growth promoters. The applied methodology was similar to the one described in the first case study.

The mid-term effects of basing a diet on the guidance provided by the present methodology are promising. For example, in a case study, after 12 weeks period of precision nutrition dietary intervention based on this food recommender method, the state of microbiome composition was significantly improved regarding general microbiome state, selected target bacteria, and specific dysbiosis described for phenotypes related to intestinal health and/or to susceptibility to specific diseases.

Sequencing results of pre and post samples showed similar technical features regarding total reads (30656 vs. 31644), taxid classifiable reads (30217 vs. 30275), and minimum relative abundance detection sensitivity (0.00014 % vs. 0.00015 %).

To ensure a reliable qualitative and quantitative determination of microbiome composition pre-and post-intervention, a sensitivity and specificity assay was carried out, across the analysis of 2 replicas of the Zymo Standard, following the same lab methodology used in problem samples.

After this procedure, the reliable sensitivity of microbiome detection was assessed in a 0.01% relative abundance with a 95% confidence interval. Therefore, only bacteria detected upon this threshold should be considered in interpreting the results (Table IV).

After the intervention, 20 new selected target bacteria were detected for the first time (10 at genus level and 10 at species level), 16 keep present (8 at genus level and 8 at species level), and 2 remain not present or undetectable upon reliable sensitivity threshold (none at genus level and 2 at species level), *Clostridium butyricum* and *Lactococcus lactis.* It is noteworthy that no targeted bacteria were lost upon this threshold after the nutritional intervention.

The mid-term effects of basing a diet on the guidance provided by this food recommender method are promising. For example, in a case study, after 12 weeks period of mainly consuming food chosen by the method, the state of microbiome composition was significantly improved regarding 1) general microbiome state, 2) selected target bacteria, and 3) phenotypic specific dysbiosis related to intestinal health and/or to susceptibility to specific diseases.

From the perspective of the overall microbiome state, an improvement was shown regarding increased biodiversity and species richness. Biodiversity gain can positively influence ecosystem functions. In the present study case, diversity was significantly enhanced, attending Margalef biodiversity index from 15.43 at first to 36.34. Similarly, a significant improvement of 119.9 % in species richness was assessed, from 166 to 365 species. Overall in ecology, a higher diversity in gut microbiome has been widely related to better health status, assuming that it implies a higher gene richness and therefore a higher allostatic potential to tolerate and survive against specific disrupting factors with the minimum wear and tear of the organism.

From the perspective of selected target bacteria, a significant enhancement was detected both at genus (from 43.24% to 94.59%) and species level (from 44.44% to 100%), on those taxa specifically related to a protective effect against intestinal permeability, inflammatory bowel disease, obesity, type II diabetes, rheumatoid arthritis, hypercholesterolemia, and kidney stones.

As indicated in Table IV, 1.3 times higher relative abundance of Bacteroides has been previously reported in patients with kidney stones disease versus control group. However, after the nutritional intervention, Bacteroides' relative abundance remained about 1%, similar to the one reported in a healthy population. Moreover, Bacteroides presence has been associate with protective effect against inflammatory bowel disease, obesity, type II diabetes, rheumatoid arthritis, and hypercholesterolemia.

It is essential to highlight the improvement detected after nutritional intervention in target bacteria regarding production of the short-chain fatty acids such as butyrate and propionate because of their pivotal role in anti-inflammatory response capacity and health in general.

Likewise, improvement was detected in the composition of probiotic bacteria, especially in those called "new generation probiotics," due to the recent discovery of their importance in human health status. This is the case of *Akkermansia muciniphila* and *Eubacterium hallii,* which only have been detected upon reliable sensitivity threshold (0.05% and 0.06%, respectively) after the food recommender method implementation. In this context, the case of *Faecalibacterium prausnitzii* is not detectable before intervention and showing a relative abundance of 59.39% after this intervention. This bacterium is one of the primary producers of butyrate and is considered one of the most critical new-generation probiotics. Furthermore, it is remarkable that *Faecalibacterium prausnitzii,* in contrast to other butyrate producers, uses inulin and pectin as substrates, both present in the specific element selected in this case by the food recommender method.

Several of these target bacteria contribute to protecting the intestinal permeability in the following coordinated way. First, *Akkermansia muciniphila* exerts mucolytic activity degrading host mucus and generating different substrates. Second, those are then used as a source of energy for non-mucus degrading bacteria with protective intestinal epithelial barrier functions, such as *Faecalibacterium prausnitzii* or *Eubacterium hallii*.

On the other hand, in the present study, an improvement has been observed in the microbiome associated with the regulation of vitamins such as B1, B2, B9, B12, and K2. It happened specifically regarding *Lactococcus* and *Lactobacillus* genera, both only detected after the nutritional intervention. Furthermore, the presence in the gut microbiome of different bacteria like Lactobacillus genera has been previously associated with an adequate regulation of neurotransmitters such as serotonin, gamma amino butyric acid, acetylcholine and dopamine.

In conclusion, this case study constitutes an example of positive mid-term effects of the food recommender method in the improvement of the general microbiome state, the relative abundance of selected target bacteria, and regarding diverse phenotypic gut dysbiosis related to intestinal health.

**Claims**

1. A computer implemented method (100) for designing a diet for a user, the method comprising the following steps:

   a) accessing (110) a database (1), the database comprising

   - a list of foods, each food comprising at least one food prebiotic, FP;
   - a first list of bacterial species commonly found in the gut microbiome of subjects from the species to which the user belongs;
   - a list of at least one bacterium prebiotic, BP, consumed by each bacterium of the first list of bacterial species; and

   b) providing (120) a second list of bacteria comprising the bacteria lacking in the user's gut microbiome and/or the bacteria present in a low quantity in the user's gut microbiome; and

   c) selecting (130) a food, F; and
   for each bacterium, $B_i$, of the second list of bacteria; wherein i is an index from 1 to the number of bacteria of said second list:

   I. obtaining (150), from the database, the list of at least one bacterium prebiotic BP consumed by the bacterium $B_i$; and
   II. comparing (160) the at least one food prebiotic FP of the food F with the list of at least one bacterium prebiotic BP, such that:

   i. if the at least one food prebiotic FP totally matches with the at least one bacterium prebiotic BP of the list, assigning (170) the food F with a maximum value predefined; or
   ii. if none of the at least one food prebiotic FP matches with the at least one bacterium prebiotic BP of the list, assigning (180) the food F with a minimum value predefined; or
   iii. if a subset of the at least one food prebiotic FP matches with the at least one bacterium prebiotic BP of the list, assigning (190) the food F with a value given by a partial sum of a harmonic series; wherein the number of elements of the harmonic series is equal to the number of food prebiotics of the subset;

   d) generating (200) a vector of growth promoting for the food F, wherein each element of the vector is the value assigned to the food F for each bacterium $B_i$ of the second list of bacteria;
   e) repeating (210) the previous steps for a plurality of foods;
   f) calculating (220), for each food, the sum of the elements of its vector of growth promoting;
   g) generating (230) a ranking of foods from the food with the highest sum of the elements of its vector of growth promoting to the food with the lowest sum of the elements of its vector of growth promoting;
   h) designing (240) the diet so that it comprises the food or foods of the top position or positions of the ranking, being the total number of foods of the diet predefined.

2. - The computer implemented method according to claim 1, wherein the database further comprises a list of at least one food consumed by at least one of the bacterium of the first list of bacterial species; and
   wherein the method further comprises the following sub-steps, being said sub-steps carried out before step c.I)

c1) obtaining (140), from the database, the list of at least one food consumed by the bacterium B$_i$, if any;

c2) if the food F is comprised in the list of at least one food consumed by the bacterium B$_i$, assigning (141) the food F with a maximum value predefined; or

c3) if the food F is not comprised in the list of at least one food consumed by the bacterium B$_i$ or if there is not a list of at least one food consumed by the bacterium B$_i$ in the database, going to step c.I) of the method.

3. - The computer implemented method according to any of the preceding claims, wherein the database further comprises a variable that defines, for each bacterium prebiotic BP, its capacity for promoting growth in the gut of each bacterium of the first list; and

wherein the value assigned to the food F in step cII.iii) is further based on said variable.

4. - The computer implemented method according to claim 3 wherein the variable is a binary variable which can have a first value that indicates that the bacterium prebiotic BP is a high growth promoter for the bacterium or a second value which indicates that the BP is a low growth promoter for the bacterium.

5. - The computer implemented method according to any of the previous claims, wherein the database further comprises the quantity in which each food prebiotic, FP, is present in each food; and

wherein the value assigned to the food F in step cII.iii) is further based on said quantities in which the at least one food prebiotic FP is present in said food F.

6. - The method according to any of the claims 3 to 5, wherein the value assigned to the food F in step cII.iii) is performed according to the following equation:

$$Value\_food(n) = \begin{cases} 0, & if\ n = 0 \\ \frac{1}{2} * max.\ value\ predefined, & if\ n = \frac{1}{2} \\ \left(\frac{1}{2} + w \sum_{i=2}^{2n} \frac{1}{i}\right) * max.\ value\ predefined, & otherwise \end{cases},$$

wherein w is a coefficient calculated as follows:

$$w = \frac{1}{2} / \left(\sum_{i=2}^{2N} \frac{1}{i}\right),$$

being $N$ the total number of bacterium prebiotics contained in the database and $n = \frac{1}{2} * n_l + 1 * n_h$, being $n_l$ the number of low-growth promoter prebiotics and $n_h$ is the number of high-growth promoter prebiotics, wherein

a low-growth promoter prebiotic is a food prebiotic that is present in the food F in a quantity below a first predefined threshold and/or a food prebiotic for which the variable that defines its capacity for promoting growth of the bacterium B$_i$ in the gut microbiome is below a second predefined threshold; and

a high-growth promoter prebiotic is a food prebiotic that is present in the food F in a quantity above or equal the first predefined threshold and/or a food prebiotic for which the variable that defines its capacity for promoting growth of the bacterium B$_i$ in the gut microbiome is above or equal to the second predefined threshold.

7. - The computer implemented method according to any of the previous claims, wherein the method further comprises the following sub-steps:

h1) calculating (241) the Euclidean distance between the vector of growth promoting of the food or foods of the diet and each of the vectors of growth promoting of the rest of the foods, if any;

h2) designing (242) the diet so that it further comprises the foods for which the distances estimated are the biggest ones, being the total number of foods of the diet predetermined.

8. - The computer implemented method according to the previous claim, wherein the estimation of the Euclidean distances of sub-step h1) is performed using an agglomerative hierarchical clustering method.

9. - The computer implemented method according to the previous claim, wherein the agglomerative hierarchical clustering method is an Unweighted Pair Group Method with Arithmetic mean (UPGMA).

10. - The computer implemented method according to any of the previous claims, wherein the value assigned to each food is normalized between zero and one.

11. - The computer implemented method according to any the previous claims, wherein the foods are selected from a group consisting of Açaí, Brown algae, Corn Hazelnuts, Onion, Sarda, Agave, Brussels sprouts, Corn bran, Honey, Passion fruit, Sea buckthorn, Almonds, Buckwheat, Cucumber, Hypericum perforatum, Peanuts, Sesame, Amaranth, Capers, Curcuma longa, Integral rice, Pear Sorghum, Apples, Carrots, Dark chocolate, Jerusalem artichoke, Peppers, Sorghum bran, Apricot, Cashew nuts, Date, Kiwi, Pineapple, Soy, Artichoke, Cassava, Echinacea purpurea, Kuding tea, Pistachio, Spelt, Asparagus, Cauliflower, Egg, Larch Plantains, Spinach, Avocado, Celery, Eggplant, Leek, Plums, Spirulina alga, Bamboo shoots, Cheese, Endives, Lentils, Pomegranate, Strawberries, Bananas, Cherries, Feijoa, Longan, Potato, Sugar cane, Barley, Chestnuts, Fermented soy milk, Lotus seeds, Pumpkin, Sunflower, Barley bran, Chia seeds, Figs, Mango, Quince, Beer, Chicken, Garlic, Milk, Quinoa, Sweet potato, Beer yeast, Chickpeas, Ginkgo biloba, Millet, Radishes, Tomato, Beetroot, Chicory, Goji, Berries, Millet bran, Raspberries, Tuna, Black tea, Citrus, Gooseberries, Mushrooms fungi, Red wine, Turkey, Blackberries, Cocoa powder, Grapes, Oat bran, Rice bran, Walnuts, Blueberries, Coconut, Green Tea, Oats, Rye, Watercress, Broad beans, Codonopsis pilosula, Green beans, Olive oil, Rye bran, Wheat, Broccoli, Coffee, Green peas, Olives, Salmon, Wheat bran, White lupin, Yacon, Yam and any combination thereof.

12. - The computer implemented method according to any the previous claims, wherein the food prebiotics and the bacterium prebiotics are from a group consisting of xylooligosaccharides (XOS), resistant starch, fructooligosaccharides (FOS), inulin, pectin, polyphenols, resveratrol, quercetin, raffinose, arabinoxylan (AX), arabinoxylan oligosaccharides (AXOS), arabinogalactans, galacto-oligosaccharides (GOS), beta-glucans, tryptophan, mannan-oligosaccharides (MOS), lignans, ellagitannins, curcumin, anthocyanins, isoflavones, and any combination thereof.

13. - The computer implemented method according to any the previous claims, wherein the bacteria of the database are selected from a group consisting of *Bacillus, Eubacterium rectale, Barnesiella, Faecalibacterium prausnitzii, Bifidobacterium, Lactobacillus acidophilus, Butyrivibrio, Lactobacillus brevis, Lachnospira, Lactobacillus delbrueckii, Leuconostoc, Lactobacillus casei, Oscillospira, Lactobacillus fermentum, Weisella, Lactobacillus paracasei, Bacillus subtilis, Lactobacillus plantarum, Bifidobacterium adolescentis, Lactobacillus reuteri, Bifidobacterium animalis, Lactobacillus rhamnosus, Bifidobacterium bifidum, Lactobacillus salivarius, Bifidobacterium longum, Leuconostoc mesenteroides, Collinsella aerofaciens, Roseburia hominis, Coprococcus catus, Roseburia inulinivorans, Enterococcus faecium, Selenomonas ruminantium, Eubacterium hallii, Streptococcus thermophiles, Anaerostipes, Anaerotruncus, Bacteroides, Butyricimonas, Gemmiger, Haemophilus, Lactococcus, Oscillibacter, Parabacteroides, Phascolarctobacterium, Subdoligranulum, Akkermansia muciniphila, Anaerostipes hadrus, Lactobacillus, Roseburia, Anaerotruncus colihominis, Bacteroides uniformis, Bacteroides vulgatus, Christensenella minuta, Clostridium butyricum, Clostridium leptum, Coprococcus, Coprococcus eutactus, Lactococcus lactis, Oxolobacter formigenes, Roseburia faecis, Roseburia intestinalis, Roseburia inulinivorans,* and any combination thereof.

14. - A data processing system comprising means for carrying out the steps of the method of any of the previous claims.

15. - A computer program comprising instructions that, when the program is executed by a computer, cause the computer to carry out the steps of the method of claims 1 to 13.

16. - A computer-readable medium comprising instructions that, when executed by a computer, cause the computer to carry out the steps of the method of claims 1 to 13.

**Patentansprüche**

1. Ein computerimplementiertes Verfahren (100) zum Entwurf einer Diät für einen Benutzer, wobei das Verfahren die folgenden Schritte umfasst:

a) Zugreifen (110) auf eine Datenbank (1), wobei die Datenbank Folgendes umfasst:

- eine Liste von Lebensmitteln, wobei jedes Lebensmittel mindestens ein Lebensmittel-Präbiotikum, FP, umfasst;
- eine erste Liste von Bakterienarten, die üblicherweise im Darmmikrobiom von Subjekten der Art gefunden werden, zu der der Benutzer gehört;
- eine Liste von mindestens einem Bakterien-Präbiotikum, BP, das von jedem Bakterium der ersten Liste von Bakterienarten konsumiert wird; und

b) Bereitstellen (120) einer zweiten Liste von Bakterien, die die Bakterien umfasst, die im Darmmikrobiom des Benutzers fehlen, und/oder die Bakterien, die in einer geringen Menge im Darmmikrobiom des Benutzers vorhanden sind; und
c) Auswählen (130) eines Lebensmittels F; und
für jedes Bakterium, $B_i$, der zweiten Liste von Bakterien; wobei i ein Index von 1 bis zur Anzahl der Bakterien dieser zweiten Liste ist:

I. Abrufen (150) aus der Datenbank der Liste von mindestens einem Bakterien-Präbiotikum BP, das von dem Bakterium $B_i$ konsumiert wird; und
II. Vergleichen (160) des mindestens einen Lebensmittel-Präbiotikums FP des Lebensmittels F mit der Liste von mindestens einem Bakterien-Präbiotikum BP, so dass:

i. wenn das mindestens eine Lebensmittel-Präbiotikum FP vollständig mit dem mindestens einen Bakterien-Präbiotikum BP der Liste übereinstimmt, dem Lebensmittel F ein vordefinierter Maximalwert zugewiesen wird (170); oder
ii. wenn keines der mindestens einen Lebensmittel-Präbiotika FP mit dem mindestens einen Bakterien-Präbiotikum BP der Liste übereinstimmt, dem Lebensmittel F ein vordefinierter Minimalwert zugewiesen wird (180); oder
iii. wenn eine Teilmenge des mindestens einen Lebensmittel-Präbiotikums FP mit dem mindestens einen Bakterien-Präbiotikum BP der Liste übereinstimmt, dem Lebensmittel F ein Wert zugewiesen wird (190), der durch eine Partialsumme einer harmonischen Reihe gegeben ist; wobei die Anzahl der Elemente der harmonischen Reihe gleich der Anzahl der Lebensmittel-Präbiotika der Teilmenge ist;

d) Erzeugen (200) eines wachstumsfördernden Vektors für das Lebensmittel F, wobei jedes Element des Vektors der Wert ist, der dem Lebensmittel F für jedes Bakterium $B_i$ der zweiten Liste von Bakterien zugewiesen wurde;
e) Wiederholen (210) der vorherigen Schritte für eine Vielzahl von Lebensmitteln;
f) Berechnen (220), für jedes Lebensmittel, der Summe der Elemente seines wachstumsfördernden Vektors;
g) Erstellen (230) einer Rangliste von Lebensmitteln, vom Lebensmittel mit der höchsten Summe der Elemente seines wachstumsfördernden Vektors bis zum Lebensmittel mit der niedrigsten Summe der Elemente seines wachstumsfördernden Vektors;
h) Entwerfen (240) der Diät so, dass sie das Lebensmittel oder die Lebensmittel der obersten Position oder Positionen der Rangliste umfasst, wobei die Gesamtanzahl der Lebensmittel der Diät vordefiniert ist.

2. - Das computerimplementierte Verfahren nach Anspruch 1, wobei die Datenbank ferner eine Liste von mindestens einem Lebensmittel umfasst, das von mindestens einem der Bakterien der ersten Liste von Bakterienarten konsumiert wird; und
wobei das Verfahren ferner die folgenden Teilschritte umfasst, wobei diese Teilschritte vor Schritt c.I) ausgeführt werden:

c1) Abrufen (140) aus der Datenbank der Liste von mindestens einem Lebensmittel, das von dem Bakterium $B_i$ konsumiert wird, falls vorhanden;
c2) wenn das Lebensmittel F in der Liste von mindestens einem Lebensmittel enthalten ist, das von dem Bakterium $B_i$ konsumiert wird, Zuweisen (141) eines vordefinierten Maximalwerts zu dem Lebensmittel F; oder
c3) wenn das Lebensmittel F nicht in der Liste von mindestens einem Lebensmittel enthalten ist, das von dem Bakterium $B_i$ konsumiert wird, oder wenn es in der Datenbank keine Liste von mindestens einem Lebensmittel gibt, das von dem Bakterium $B_i$ konsumiert wird, Weitergehen zu Schritt c.I) des Verfahrens.

3. - Das computerimplementierte Verfahren nach einem der vorhergehenden Ansprüche, wobei die Datenbank ferner eine Variable umfasst, die für jedes Bakterien-Präbiotikum BP seine Kapazität zur Förderung des Wachstums im Darm jedes Bakteriums der ersten Liste definiert; und
wobei der in Schritt cII.iii) dem Lebensmittel F zugewiesene Wert ferner auf dieser Variablen basiert.

4. - Das computerimplementierte Verfahren nach Anspruch 3, wobei die Variable eine binäre Variable ist, die einen ersten Wert haben kann, der anzeigt, dass das Bakterien-Präbiotikum BP ein starker Wachstumsförderer für das Bakterium ist, oder einen zweiten Wert, der anzeigt, dass das BP ein schwacher Wachstumsförderer für das Bakterium ist.

**5.** - Das computerimplementierte Verfahren nach einem der vorhergehenden Ansprüche, wobei die Datenbank ferner die Menge umfasst, in der jedes Lebensmittel-Präbiotikum FP in jedem Lebensmittel vorhanden ist; und wobei der in Schritt cII.iii) dem Lebensmittel F zugewiesene Wert ferner auf diesen Mengen basiert, in denen das mindestens eine Lebensmittel-Präbiotikum FP in dem Lebensmittel F vorhanden ist.

**6.** - Das Verfahren nach einem der Ansprüche 3 bis 5, wobei der dem Lebensmittel F in Schritt cII.iii) zugewiesene Wert gemäß der folgenden Gleichung bestimmt wird:

$$Wert\_Lebensmittel(n) = \begin{cases} 0, & wenn\ n = 0 \\ \frac{1}{2} * vordefinierter\ Maximalwert, & wenn\ n = \frac{1}{2} \\ \left(\frac{1}{2} + w \sum_{i=2}^{2n} \frac{1}{i}\right) * vordefinierter\ Maximalwert, & andernfalls \end{cases},$$

wobei $w$ ein Koeffizient ist, der wie folgt berechnet wird:

$$w = \frac{1}{2} / \left(\sum_{i=2}^{2N} \frac{1}{i}\right),$$

wobei $N$ die Gesamtanzahl der Bakterien-Präbiotika in der Datenbank ist und $n = \frac{1}{2} * n_l + 1 * n_h$, wobei $n_l$ die Anzahl der schwachen Wachstumsförderer und $n_h$ die Anzahl der starken Wachstumsförderer ist, wobei

ein Präbiotikum mit schwacher Wachstumsförderung ein Lebensmittel-Präbiotikum ist, das in dem Lebensmittel F in einer Menge unterhalb eines ersten vordefinierten Schwellenwerts vorhanden ist, und/oder ein Lebensmittel-Präbiotikum, für das die Variable, die seine Kapazität zur Förderung des Wachstums des Bakteriums $B_i$ im Darmmikrobiom definiert, unterhalb eines zweiten vordefinierten Schwellenwerts liegt; und ein Präbiotikum mit starker Wachstumsförderung ein Lebensmittel-Präbiotikum ist, das in dem Lebensmittel F in einer Menge oberhalb oder gleich dem ersten vordefinierten Schwellenwert vorhanden ist, und/oder ein Lebensmittel-Präbiotikum, für das die Variable, die seine Kapazität zur Förderung des Wachstums des Bakteriums $B_i$ im Darmmikrobiom definiert, oberhalb oder gleich dem zweiten vordefinierten Schwellenwert liegt.

**7.** - Das computerimplementierte Verfahren nach einem der vorhergehenden Ansprüche, wobei das Verfahren ferner die folgenden Teilschritte umfasst:

h1) Berechnen (241) des euklidischen Abstands zwischen dem wachstumsfördernden Vektor des Lebensmittels oder der Lebensmittel der Diät und jedem der wachstumsfördernden Vektoren der übrigen Lebensmittel, falls vorhanden;
h2) Entwerfen (242) der Diät so, dass sie ferner die Lebensmittel umfasst, für die die geschätzten Abstände am größten sind, wobei die Gesamtanzahl der Lebensmittel der Diät vorbestimmt ist.

**8.** - Das computerimplementierte Verfahren nach dem vorhergehenden Anspruch, wobei die Schätzung der euklidischen Abstände aus Teilschritt h1) unter Verwendung eines agglomerativen hierarchischen Clustering-Verfahrens durchgeführt wird.

**9.** - Das computerimplementierte Verfahren nach dem vorhergehenden Anspruch, wobei das agglomerative hierarchische Clustering-Verfahren ein ungewichtetes Paargruppenverfahren mit arithmetischem Mittelwert (UPGMA) ist.

**10.** - Das computerimplementierte Verfahren nach einem der vorhergehenden Ansprüche, wobei der jedem Lebensmittel zugewiesene Wert zwischen null und eins normiert ist.

**11.** - Das computerimplementierte Verfahren nach einem der vorhergehenden Ansprüche, wobei die Lebensmittel ausgewählt sind aus einer Gruppe bestehend aus Acai, Braunalgen, Mais, Haselnüssen, Zwiebeln, Sarda, Agave, Rosenkohl, Maiskleie, Honig, Passionsfrucht, Sanddorn, Mandeln, Buchweizen, Gurke, Hypericum perforatum, Erdnüssen, Sesam, Amaranth, Kapern, Curcuma longa, Vollkornreis, Birne, Sorghum, Äpfeln, Karotten, dunkler Schokolade, Topinambur, Paprika, Sorghumkleie, Aprikose, Cashewnüssen, Dattel, Kiwi, Ananas, Soja, Artischocke, Maniok, Echinacea purpurea, Kuding-Tee, Pistazie, Dinkel, Spargel, Blumenkohl, Ei, Lärche, Kochbananen, Spinat, Avocado, Sellerie, Aubergine, Lauch, Pflaumen, Spirulina-Alge, Bambussprossen, Käse, Endivien, Linsen, Granatapfel, Erdbeeren, Bananen, Kirschen, Feijoa, Longan, Kartoffel, Zuckerrohr, Gerste, Kastanien, fermentierter Sojamilch, Lotussamen, Kürbis, Sonnenblume, Gerstenkleie, Chiasamen, Feigen, Mango, Quitte, Bier, Hühnchen, Knoblauch, Milch, Quinoa, Süßkartoffel, Bierhefe, Kichererbsen, Ginkgo biloba, Hirse, Radieschen, Tomate, Roter Bete, Zichorie, Goji-Beeren, Hirsekleie, Himbeeren, Thunfisch, Schwarztee, Zitrusfrüchten, Stachelbeeren, Pilzen, Rotwein, Truthahn, Brombeeren, Kakaopulver, Trauben, Haferkleie, Reiskleie, Walnüssen, Blaubeeren, Kokosnuss, Grüntee, Hafer, Roggen, Brunnenkresse, Ackerbohnen, Codonopsis pilosula, grünen Bohnen, Olivenöl, Roggenkleie, Weizen, Brokkoli, Kaffee, grünen Erbsen, Oliven, Lachs, Weizenkleie, weißer Lupine, Yacon, Yamswurzel und einer beliebigen Kombination davon.

12. - Das computerimplementierte Verfahren nach einem der vorhergehenden Ansprüche, wobei die Lebensmittel-Präbiotika und die Bakterien-Präbiotika ausgewählt sind aus einer Gruppe bestehend aus Xylooligosacchariden (XOS), resistenter Stärke, Fructooligosacchariden (FOS), Inulin, Pektin, Polyphenolen, Resveratrol, Quercetin, Raffinose, Arabinoxylan (AX), Arabinoxylan-Oligosacchariden (AXOS), Arabinogalactanen, Galacto-Oligosacchariden (GOS), Beta-Glucanen, Tryptophan, Mannan-Oligosacchariden (MOS), Lignanen, Ellagitanninen, Curcumin, Anthocyanen, Isoflavonen und einer beliebigen Kombination davon.

13. - Das computerimplementierte Verfahren nach einem der vorhergehenden Ansprüche, wobei die Bakterien der Datenbank ausgewählt sind aus einer Gruppe bestehend aus *Bacillus, Eubacterium rectale, Barnesiella, Faecalibacterium prausnitzii, Bifidobacterium, Lactobacillus acidophilus, Butyrivibrio, Lactobacillus brevis, Lachnospira, Lactobacillus delbrueckii, Leuconostoc, Lactobacillus casei, Oscillospira, Lactobacillus fermentum, Weisella, Lactobacillus paracasei, Bacillus subtilis, Lactobacillus plantarum, Bifidobacterium adolescentis, Lactobacillus reuteri, Bifidobacterium animalis, Lactobacillus rhamnosus, Bifidobacterium bifidum, Lactobacillus salivarius, Bifidobacterium longum, Leuconostoc mesenteroides, Collinsella aerofaciens, Roseburia hominis, Coprococcus catus, Roseburia inulinivorans, Enterococcus faecium, Selenomonas ruminantium, Eubacterium hallii, Streptococcus thermophiles, Anaerostipes, Anaerotruncus, Bacteroides, Butyricimonas, Gemmiger, Haemophilus, Lactococcus, Oscillibacter, Parabacteroides, Phascolarctobacterium, Subdoligranulum, Akkermansia muciniphila, Anaerostipes hadrus, Lactobacillus, Roseburia, Anaerotruncus colihominis, Bacteroides uniformis, Bacteroides vulgatus, Christensenella minuta, Clostridium butyricum, Clostridium leptum, Coprococcus, Coprococcus eutactus, Lactococcus lactis, Oxolobacter formigenes, Roseburia faecis, Roseburia intestinalis, Roseburia inulinivorans* und einer beliebigen Kombination davon.

14. - Ein Datenverarbeitungssystem, umfassend Mittel zur Ausführung der Schritte des Verfahrens nach einem der vorhergehenden Ansprüche.

15. - Ein Computerprogramm, umfassend Befehle, die bewirken, dass der Computer die Schritte des Verfahrens nach den Ansprüchen 1 bis 13 ausführt, wenn das Programm von einem Computer ausgeführt wird.

16. - Ein computerlesbares Medium, umfassend Befehle, die bewirken, dass der Computer die Schritte des Verfahrens nach den Ansprüchen 1 bis 13 ausführt, wenn sie von einem Computer ausgeführt werden.

**Revendications**

1. Un procédé mis en œuvre par ordinateur (100) pour concevoir un régime alimentaire pour un utilisateur, le procédé comprenant les étapes suivantes :

a) accéder (110) à une base de données (1), la base de données comprenant

- une liste d'aliments, chaque aliment comprenant au moins un prébiotique alimentaire, FP ;
- une première liste d'espèces bactériennes couramment trouvées dans le microbiote intestinal de sujets de l'espèce à laquelle appartient l'utilisateur ;
- une liste d'au moins un prébiotique bactérien, BP, consommé par chaque bactérie de la première liste d'espèces bactériennes ; et

b) fournir (120) une deuxième liste de bactéries comprenant les bactéries absentes du microbiote intestinal de l'utilisateur et/ou les bactéries présentes en faible quantité dans le microbiote intestinal de l'utilisateur ; et

c) sélectionner (130) un aliment, F ; et

pour chaque bactérie, $B_i$, de la deuxième liste de bactéries ; dans laquelle i est un index de 1 au nombre de bactéries de ladite deuxième liste :

I. obtenir (150), à partir de la base de données, la liste d'au moins un prébiotique bactérien BP consommé par la bactérie $B_i$ ; et

II. comparer (160) l'au moins un prébiotique alimentaire FP de l'aliment F avec la liste d'au moins un prébiotique bactérien BP, de sorte que :

i. si l'au moins un prébiotique alimentaire FP correspond totalement à l'au moins un prébiotique bactérien BP de la liste, attribuer (170) à l'aliment F une valeur maximale prédéfinie ; ou

ii. si aucun de l'au moins un prébiotique alimentaire FP ne correspond à l'au moins un prébiotique bactérien BP de la liste, attribuer (180) à l'aliment F une valeur minimale prédéfinie ; ou

iii. si un sous-ensemble de l'au moins un prébiotique alimentaire FP correspond à l'au moins un prébiotique bactérien BP de la liste, attribuer (190) à l'aliment F une valeur donnée par une somme partielle d'une série harmonique ; dans laquelle le nombre d'éléments de la série harmonique est égal au nombre de prébiotiques alimentaires du sous-ensemble ;

d) générer (200) un vecteur de promotion de la croissance pour l'aliment F, dans lequel chaque élément du vecteur est la valeur attribuée à l'aliment F pour chaque bactérie Bi de la deuxième liste de bactéries ;

e) répéter (210) les étapes précédentes pour une pluralité d'aliments ;

f) calculer (220), pour chaque aliment, la somme des éléments de son vecteur de promotion de la croissance ;

g) générer (230) un classement des aliments, de l'aliment ayant la somme la plus élevée des éléments de son vecteur de promotion de la croissance à l'aliment ayant la somme la plus faible des éléments de son vecteur de promotion de la croissance ;

h) concevoir (240) le régime alimentaire de sorte qu'il comprenne l'aliment ou les aliments de la ou des premières positions du classement, le nombre total d'aliments du régime étant prédéfini.

2. - Le procédé mis en œuvre par ordinateur selon la revendication 1, dans lequel la base de données comprend en outre une liste d'au moins un aliment consommé par au moins une des bactéries de la première liste d'espèces bactériennes ; et

dans lequel le procédé comprend en outre les sous-étapes suivantes, lesdites sous-étapes étant effectuées avant l'étape c.I)

c1) obtenir (140), à partir de la base de données, la liste d'au moins un aliment consommé par la bactérie $B_i$, le cas échéant ;

c2) si l'aliment F est compris dans la liste d'au moins un aliment consommé par la bactérie $B_i$, attribuer (141) à l'aliment F une valeur maximale prédéfinie ; ou

c3) si l'aliment F n'est pas compris dans la liste d'au moins un aliment consommé par la bactérie $B_i$ ou s'il n'y a pas de liste d'au moins un aliment consommé par la bactérie $B_i$ dans la base de données, passer à l'étape c.I) du procédé.

3. - Le procédé mis en œuvre par ordinateur selon l'une quelconque des revendications précédentes, dans lequel la base de données comprend en outre une variable qui définit, pour chaque prébiotique bactérien BP, sa capacité à promouvoir la croissance dans l'intestin de chaque bactérie de la première liste ; et

dans lequel la valeur attribuée à l'aliment F à l'étape cII.iii) est en outre basée sur ladite variable.

4. - Le procédé mis en œuvre par ordinateur selon la revendication 3, dans lequel la variable est une variable binaire qui peut avoir une première valeur indiquant que le prébiotique bactérien BP est un promoteur de croissance élevé pour la bactérie ou une deuxième valeur indiquant que le BP est un promoteur de croissance faible pour la bactérie.

5. - Le procédé mis en œuvre par ordinateur selon l'une quelconque des revendications précédentes, dans lequel la base de données comprend en outre la quantité dans laquelle chaque prébiotique alimentaire, FP, est présent dans chaque aliment ; et

dans lequel la valeur attribuée à l'aliment F à l'étape cII.iii) est en outre basée sur lesdites quantités dans lesquelles l'au moins un prébiotique alimentaire FP est présent dans ledit aliment F.

**6.** - Le procédé selon l'une quelconque des revendications 3 à 5, dans lequel la valeur attribuée à l'aliment F à l'étape cII.iii) est effectuée selon l'équation suivante :

$$Valeur\_aliment(n) = \begin{cases} 0, & si\ n = 0 \\ \frac{1}{2} * valeur\ max.\ prédéfinie, & si\ n = \frac{1}{2}, \\ \left(\frac{1}{2} + w\sum_{i=2}^{2n}\frac{1}{i}\right) * valeur\ max.\ prédéfinie, & sinon \end{cases}$$

dans laquelle w est un coefficient calculé comme suit :

$$w = \frac{1}{2} / \left(\sum_{i=2}^{2N}\frac{1}{i}\right),$$

avec $N$ étant le nombre total de prébiotiques bactériens contenus dans la base de données et $n = \frac{1}{2} * n_l + 1 * n_h$, $n_l$ étant le nombre de prébiotiques promoteurs de croissance faible et $n_h$ est le nombre de prébiotiques promoteurs de croissance élevée, dans lequel

un prébiotique à faible promotion de croissance est un prébiotique alimentaire qui est présent dans l'aliment F en une quantité inférieure à un premier seuil prédéfini, et/ou un prébiotique alimentaire pour lequel la variable qui définit sa capacité à promouvoir la croissance de la bactérie $B_i$ dans le microbiome intestinal est inférieure à un second seuil prédéfini ; et
un prébiotique à forte promotion de croissance est un prébiotique alimentaire qui est présent dans l'aliment F en une quantité supérieure ou égale au premier seuil prédéfini, et/ou un prébiotique alimentaire pour lequel la variable qui définit sa capacité à promouvoir la croissance de la bactérie $B_i$ dans le microbiome intestinal est supérieure ou égale au second seuil prédéfini.

**7.** - Le procédé mis en œuvre par ordinateur selon l'une quelconque des revendications précédentes, dans lequel le procédé comprend en outre les sous-étapes suivantes :

h1) calculer (241) la distance euclidienne entre le vecteur de promotion de la croissance de l'aliment ou des aliments du régime et chacun des vecteurs de promotion de la croissance des autres aliments, le cas échéant ;
h2) concevoir (242) le régime de sorte qu'il comprenne en outre les aliments pour lesquels les distances estimées sont les plus grandes, le nombre total d'aliments du régime étant prédéterminé.

**8.** - Le procédé mis en œuvre par ordinateur selon la revendication précédente, dans lequel l'estimation des distances euclidiennes de la sous-étape h1) est effectuée en utilisant une méthode de regroupement hiérarchique aggloméra-rative.

**9.** - Le procédé mis en œuvre par ordinateur selon la revendication précédente, dans lequel la méthode de regroupement hiérarchique agglomérative est une méthode de groupe de paires non pondérées avec moyenne arithmétique (UPGMA).

**10.** - Le procédé mis en œuvre par ordinateur selon l'une quelconque des revendications précédentes, dans lequel la valeur attribuée à chaque aliment est normalisée entre zéro et un.

**11.** - Le procédé mis en œuvre par ordinateur selon l'une quelconque des revendications précédentes, dans lequel les aliments sont sélectionnés dans un groupe constitué de açaï, algues brunes, maïs, noisettes, oignon, sarda, agave, choux de Bruxelles, son de maïs, miel, fruit de la passion, argousier, amandes, sarrasin, concombre, millepertuis (Hypericum perforatum), arachides, sésame, amarante, câpres, curcuma (Curcuma longa), riz complet, poire, sorgho, pommes, carottes, chocolat noir, topinambour, poivrons, son de sorgho, abricot, noix de cajou, datte, kiwi, ananas, soja, artichaut, manioc, échinacée (Echinacea purpurea), thé Kuding, pistache, épeautre, asperge, chou-fleur, œuf, mélèze, bananes plantains, épinards, avocat, céleri, aubergine, poireau, prunes, algue spiruline, pousses de bambou, fromage, endives, lentilles, grenade, fraises, bananes, cerises, feijoa, longane, pomme de terre, canne à sucre, orge, châtaignes, lait de soja fermenté, graines de lotus, citrouille, tournesol, son d'orge, graines de chia, figues, mangue, coing, bière, poulet, ail, lait, quinoa, patate douce, levure de bière, pois chiches, ginkgo biloba, millet, radis, tomate, betterave, chicorée, goji, baies, son de millet, framboises, thon, thé noir, agrumes, groseilles, champignons, vin rouge, dinde, mûres, poudre de cacao, raisins, son d'avoine, son de riz, noix, myrtilles, noix de coco, thé vert, avoine, seigle, cresson, fèves, codonopsis (Codonopsis pilosula), haricots verts, huile d'olive, son de seigle, blé, brocoli, café, petits pois, olives, saumon, son de blé, lupin blanc, yacon, igname et toute combinaison de ceux-ci.

**12.** - Le procédé mis en œuvre par ordinateur selon l'une quelconque des revendications précédentes, dans lequel les prébiotiques alimentaires et les prébiotiques bactériens proviennent d'un groupe constitué de xylooligosaccharides (XOS), amidon résistant, fructooligosaccharides (FOS), inuline, pectine, polyphénols, resvératrol, quercétine, raffinose, arabinoxylane (AX), oligosaccharides d'arabinoxylane (AXOS), arabinogalactanes, galacto-oligosaccharides (GOS), bêta-glucanes, tryptophane, mannanoligosaccharides (MOS), lignanes, ellagitanins, curcumine, anthocyanes, isoflavones et toute combinaison de ceux-ci.

**13.** - Le procédé mis en œuvre par ordinateur selon l'une quelconque des revendications précédentes, dans lequel les bactéries de la base de données sont sélectionnées dans un groupe constitué de *Bacillus, Eubacterium rectale, Barnesiella, Faecalibacterium prausnitzii, Bifidobacterium, Lactobacillus acidophilus, Butyrivibrio, Lactobacillus brevis, Lachnospira, Lactobacillus delbrueckii, Leuconostoc, Lactobacillus casei, Oscillospira, Lactobacillus fermentum, Weisella, Lactobacillus paracasei, Bacillus subtilis, Lactobacillus plantarum, Bifidobacterium adolescentis, Lactobacillus reuteri, Bifidobacterium animalis, Lactobacillus rhamnosus, Bifidobacterium bifidum, Lactobacillus salivarius, Bifidobacterium longum, Leuconostoc mesenteroides, Collinsella aerofaciens, Roseburia hominis, Coprococcus catus, Roseburia inulinivorans, Enterococcus faecium, Selenomonas ruminantium, Eubacterium hallii, Streptococcus thermophiles, Anaerostipes, Anaerotruncus, Bacteroides, Butyricimonas, Gemmiger, Haemophilus, Lactococcus, Oscillibacter, Parabacteroides, Phascolarctobacterium, Subdoligranulum, Akkermansia muciniphila, Anaerostipes hadrus, Lactobacillus, Roseburia, Anaerotruncus colihominis, Bacteroides uniformis, Bacteroides vulgatus, Christensenella minuta, Clostridium butyricum, Clostridium leptum, Coprococcus, Coprococcus eutactus, Lactococcus lactis, Oxolobacter formigenes, Roseburia faecis, Roseburia intestinalis, Roseburia inulinivorans,* et toute combinaison de ceux-ci.

**14.** - Un système de traitement de données comprenant des moyens pour effectuer les étapes du procédé selon l'une quelconque des revendications précédentes.

**15.** - Un programme informatique comprenant des instructions qui, lorsque le programme est exécuté par un ordinateur, amènent l'ordinateur à effectuer les étapes du procédé des revendications 1 à 13.

**16.** - Un support lisible par ordinateur comprenant des instructions qui, lorsqu'elles sont exécutées par un ordinateur, amènent l'ordinateur à effectuer les étapes du procédé des revendications 1 à 13.

```
                    ┌─────────┐
                    │   110   │
                    └─────────┘
                         │
                         ▼
                    ┌─────────┐
                    │   120   │
                    └─────────┘
                         │
                         ▼
                    ┌─────────┐
                    │   130   │
                    └─────────┘
                         │
    ┌────────────────────┼──────────────────────────────────┐
    │                    ▼                                   │
    │               ┌─────────┐                              │
    │               │   150   │                              │
    │               └─────────┘                              │
    │                    │                                   │
    │    ⟲ Bi             ▼                                   │
    │               ┌─────────┐                              │
    │               │   160   │                              │
    │               └─────────┘                              │
    │                    │                                   │
    │                          yes   ┌─────────┐             │
    │          FP = BP?  ──────────▶ │   170   │───┐         │
    │                                └─────────┘   │         │
    │                     no                       │         │
    │                          yes   ┌─────────┐   │         │
    │          FP ≠ BP?  ──────────▶ │   180   │───┤         │
    │                                └─────────┘   │         │
    │                     no                       │         │
    │                                ┌─────────┐   │         │
    │          otherwise ──────────▶ │   190   │───┤         │
    │                                └─────────┘   │         │
    └──────────────────────────────────────────────┼────────┘
                    ┌─────────┐                     │
                    │   200   │◀────────────────────┘
                    └─────────┘
                         │
                         ▼
                    ┌─────────┐
                    │   210   │
                    └─────────┘
                         │
                         ▼
                    ┌─────────┐
                    │   220   │
                    └─────────┘
                         │
                         ▼
                    ┌─────────┐
                    │   230   │
                    └─────────┘
                         │
                         ▼
                    ┌─────────┐
                    │   240   │
                    └─────────┘
```

Fig. 1a

```
                        ┌─────────┐
                        │   110   │
                        └─────────┘
                             │
                             ▼
                        ┌─────────┐
                        │   120   │
                        └─────────┘
                             │
                             ▼
                        ┌─────────┐
                        │   130   │
                        └─────────┘
                             │
  ┌ ─ ─ ─ ─ ─ ─ ─ ─ ─ ─ ─ ─ ─│─ ─ ─ ─ ─ ─ ─ ─ ─ ─ ─ ─ ─ ─ ┐
  │                          ▼                             │
  │                     ┌─────────┐                        │
  │                     │   140   │                        │
  │                     └─────────┘                        │
  │                                                        │
  │                            yes       ┌─────────┐        │
  │         Bi consumes F? ───────────▶  │   141   │        │
  │                          │           └─────────┘        │
  │                          no                             │
  │                          ▼                              │
  │                     ┌─────────┐                         │
  │                     │   150   │                         │
  │    ╭──╮             └─────────┘                         │
  │   ╱   ╲   Bi             │                              │
  │  ╰────╯╮                 ▼                              │
  │        ╲           ┌─────────┐                          │
  │                    │   160   │                          │
  │                    └─────────┘                          │
  │                                                         │
  │                          yes      ┌─────────┐           │
  │          FP = BP? ──────────────▶ │   170   │──┐        │
  │                   │               └─────────┘  │        │
  │                   no                           │        │
  │                             yes   ┌─────────┐  │        │
  │          FP ≠ BP? ──────────────▶ │   180   │──┤        │
  │                   │               └─────────┘  │        │
  │                   no                           │        │
  │                             yes   ┌─────────┐  │        │
  │          otherwise ─────────────▶ │   190   │──┤        │
  │                                   └─────────┘  │        │
  └ ─ ─ ─ ─ ─ ─ ─ ─ ─ ─ ─ ─ ─ ─ ─ ─ ─ ─ ─ ─ ─ ─ ─│─ ─ ─ ─ ┘
                                                   │
                        ┌─────────┐                │
                        │   200   │◀───────────────┘
                        └─────────┘
                             │
                             ▼
                        ┌─────────┐
                        │   210   │
                        └─────────┘
                             │
                             ▼
                        ┌─────────┐
                        │   220   │
                        └─────────┘
                             │
                             ▼
                        ┌─────────┐
                        │   230   │
                        └─────────┘
                             │
                             ▼
                        ┌─────────┐
                        │   240   │
                        └─────────┘
```

# Fig. 1b

Fig. 1c

Fig. 2

Fig. 3

Fig. 4

Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020177518 B

- US 2020381089 A1

**Non-patent literature cited in the description**

- **KAI, S. et al.** Rapid bacterial identification by direct PCR amplification of 16S rRNA genes using the MinION™ nanopore sequencer. *FEBS Open Bio*, 2019, vol. 9, 548-557

- **STODDARD, S. F.** ; **SMITH, B. J.** ; **HEIN, R.** ; **ROLLER, B. R. K.** ; **SCHMIDT, T. M.** rrnDB: improved tools for interpreting rRNA gene abundance in bacteria and archaea and a new foundation for future development. *Nucleic Acids Res*, 2015, vol. 43, D593-D598
- **ZEAITER, M. E. et al.** *BioMed research international*, vol. 2019